# EUROPÄISCHE PATENTSCHRIFT

(11) **EP 3 182 957 B1**
(45) Veröffentlichungstag und Bekanntmachung des Hinweises auf die Patenterteilung: **16.10.2019**
(21) Anmeldenummer: 15733408.7
(22) Anmeldetag: 25.06.2015
(51) Int. Cl.: A61P 11/02, A61K 9/00, A61K 47/34, A61K 47/36, A61K 31/164, A61K 31/352, A61K 47/02, A61K 47/18, A61K 47/44, A61K 31/197, A61K 31/4174, A61K 31/505

(54) **CINEOLHALTIGE ZUSAMMENSETZUNG FÜR DIE NASALE APPLIKATION**
CINEOLE-CONTAINING COMPOSITION FOR NASAL APPLICATION
COMPOSITION CONTENANT DU CINÉOL POUR APPLICATION NASALE

(30) Priorität: 18.08.2014 DE 102014012040; 22.08.2014 DE 102014012278; 10.09.2014 DE 102014013145; 11.09.2014 DE 102014013189; 19.11.2014 DE 102014116903
(43) Veröffentlichungstag der Anmeldung: 28.06.2017
(60) Teilanmeldung: 17203038.9
(73) Patentinhaber: Maria Clementine Martin Klosterfrau Vertriebsgesellschaft mbH, 50670 Köln (DE)
(72) Erfinder: UNKAUF, Markus, 80538 München (DE)
(74) Vertreter: Von Rohr Patentanwälte Partnerschaft mbB
(86) Internationale Anmeldenummer: PCT/EP2015/064373
(87) Internationale Veröffentlichungsnummer: WO 2016/026600

(56) Entgegenhaltungen:
- EP-B1- 2 320 870
- DE-A1-102013 001 151
- DE-U1-202012 002 792
- US-A1- 2004 081 699

## Beschreibung

Die vorliegende Erfindung betrifft das technische (d. h. medizinisch-therapeutische) Gebiet der Behandlung von Rhinitiden.

Insbesondere betrifft die vorliegende Erfindung eine cineolhaltige Zusammensetzung, insbesondere eine cineolhaltige pharmazeutische Zusammensetzung, welche sich vorzugsweise für die topische, insbesondere nasale, bevorzugt intranasale Applikation eignet, insbesondere für die Behandlung von Rhinitiden, und die Verwendung dieser cineolhaltigen Zusammensetzung sowie eine diese cineolhaltige Zusammensetzung enthaltende Applikationsvorrichtung. Die erfindungsgemäße cineolhaltige Zusammensetzung wird nachfolgend gelegentlich synonym auch als erfinderische Kombination bzw. erfinderische Wirkstoffkombination oder als erfinderisches Antirhinitismittel (Antirhinitikum) bezeichnet.

Unter einer Rhinitis (synonym gelegentlich auch als Nasenkatarrh, Schnupfen oder Koryza bezeichnet) versteht man im Rahmen der vorliegenden Erfindung insbesondere eine akute oder chronische Entzündung der Nasenschleimhäute, wobei die Rhinitis insbesondere infektiösen (z. B. viralen oder bakteriellen), allergischen oder pseudoallergischen Ursprungs sein kann. Am Häufigsten tritt eine Rhinitis im Rahmen einer sogenannten Erkältung auf.

Neben einer Unterscheidung von akuter Rhinitis einerseits und chronischer Rhinitis andererseits unterscheidet man auch verschiedene Formen von Rhinitiden, so beispielsweise die folgenden Rhinitisformen: *Rhinitis acuta, Rhinitis atrophicans, Rhinitis allergica, Rhinitis hypertrophica, Rhinitis medicamentosa, Rhinitis pseudomembranacea, Rhinitis sicca, Rhinitis vasomotorica* und die umweltbedingte Rhinitis.

Bei der sogenannten akuten Rhinitis (*Rhinitis acuta*), d. h. dem gewöhnlichen Schnupfen, handelt es sich in der Regel um einen im Allgemeinen harmlosen Infekt der Nasenschleimhäute und damit um eine infektiöse Rhinitis, welche meist viral, insbesondere durch eine Vielzahl von Viren (insbesondere Rhinoviren und/oder Adenoviren), gelegentlich aber auch bakteriell ausgelöst werden kann. Hauptmerkmal einer akuten Rhinitis ist eine sogenannte laufende Nase und eine Verstopfung der Nase durch die Anschwellung der Schleimhäute.

Insgesamt sind mehr als 200 "Schnupfenviren" als mögliche Auslöser einer viralen Rhinitis bekannt, wie sie üblicherweise im Rahmen einer gewöhnlichen Erkältung auftreten kann. Im Rahmen einer Erkältung, welche im Allgemeinen mit einer Rhinitis beginnt, verschwindet aber die *Rhinitis acuta* im Allgemeinen. Gelegentlich kann es jedoch mitunter auch zu einer Chronifizierung kommen, welche oftmals mit einer Volumenzunahme der Schleimhäute unter anderem im Bereich der Nasenmuscheln mit Behinderung der Nasenatmung einhergeht.

Eine Rhinitis (d. h. eine Entzündung der Nasenschleimhaut), insbesondere eine akute Rhinitis, kann oftmals gleichzeitig auch mit einer Entzündung der Schleimhaut der Nasennebenhöhlen (Sinusitis) vorliegen. In einem solchen Fall spricht man auch von einer Rhinosinusitis.

Für weitergehende Einzelheiten zum Begriff der Rhinitis kann insbesondere verwiesen werden auf Pschyrembel, Medizinisches Wörterbuch, 257. Auflage, Seiten 1331/1332, insbesondere Stichwörter: *"Rhinitis", "Rhinitis allergica", "Rhinitis atrophicans", "Rhinitis hyperplastica", "Rhinitis pseudomembranacea", "Rhinitis sicca"* und *"Rhinitis vasomotorica".*

Da es also eine Vielzahl verschiedener Typen von Viren gibt, welche eine akute virale Rhinitis auslösen können, und da eine Vielzahl von Ursachen für das Auftreten einer Rhinitis existieren, können Rhinitiden, insbesondere akute Rhinitiden, im Allgemeinen nicht kausal, sondern nur symptomatisch, insbesondere topisch, behandelt werden.

Zu diesem Zweck kommen in den meisten Fällen intranasal zu applizierende Zusammensetzungen zur Anwendung, welche sogenannte Sympathomimetika (synonym auch als "Abschweller", "Dekongestiva" oder dergleichen bezeichnet), vorzugweise alpha-Sympathomimetika (wie z. B. Xylometazolin und Oxymetazolin oder deren physiologisch verträgliche bzw. unbedenkliche Salze), enthalten.

Diese Sympathomimetika führen zwar aufgrund ihrer vasokonstriktorischen Eigenschaften nach lokaler bzw. topischer Anwendung in der Nase zunächst zu einer Nasenschleimhautabschwellung, bewirken jedoch bei wiederholten Anwendung oftmals eine Austrocknung der Nasenschleimhäute, einhergehend mit entzündlichen Irritationen der Nasenschleimhäute (was nicht selten zu einer erhöhten Infektionsgefahr führt, da die Nasenschleimhaut im ausgetrockneten und entzündeten Zustand nicht mehr ihre Schutz- und Filterfunktion im vollen Umfang aufrechterhalten kann und folglich Krankheitserreger ungehindert in die Atemwege gelangen können).

Darüber hinaus wirken die Sympathomimetika weder antibakteriell noch antiviral. Auch haben die Sympathomimetika zum Teil auch schwerwiegende systemische (z. B. kardiovaskuläre) Nebenwirkungen, insbesondere im Fall von Überdosierung oder bei der Therapierung bestimmter Patienten (z. B. Kleinkindern, Patienten mit kardiovaskulären Vorerkrankungen etc.).

Um den zahlreichen Nebenwirkungen der Sympathomimetika zumindest teilweise entgegenzuwirken und um das zum Teil unzulängliche Wirkprofil der Sympathomimetika bei der Behandlung von Rhinitiden zumindest in gewisser Weise zu verbessern, werden in für die intranasale Applikation bestimmte sympathomimetikahaltigen Zusammensetzungen bisweilen weitere Wirk- bzw. Inhaltsstoffe inkorporiert, beispielsweise pflanzliche Extrakte oder pflanzliche Inhaltsstoffe, befeuchtende oder schleimhautpflegende Zusätze, Antihistaminika, Salze etc. (vgl. beispielsweise DE 195 41 919 A1, DE 195 49 421 A1 und DE 103 56 248 A1).

Weiterhin beschreibt die EP 2 320 870 B1 eine topische intranasale Zusammensetzung zur Verwendung bei Nasenverstopfungen, wobei die Zusammensetzung Carboxymethyl-β-glukan bzw. Phophoglukan mit 18α- bzw. β-Oycyrrhetinsäure und Lactoferrin zur Verhinderung der nasalen Verstopfung enthält. Darüber hinaus kann die Zusammensetzung neben einer Vielzahl weiterer ätherischer Öle auch Eukalyptus bzw. natürliches Eukalyptusaroma enthalten.

Die DE 10 2013 001 151 A1 betrifft eine Zubereitung zur topischen nasalen Anwendung auf Schleimhäuten auf Basis von Natriumchlorid-Salz in wässriger Lösung, wobei die Zusammensetzung zudem einen Alkohol, ein Poloxamer-Tensid sowie gegebenenfalls weitere Zusatzstoffe aufweisen soll. In diesem Zusammenhang kann es vorgesehen sein, dass die Zusammensetzung neben Vitaminen, pH-Wert-Regulatoren und Konservierungsmitteln auch ätherische Öle aufweist, wobei neben einer Vielzahl an ätherischen Ölen auch Eukalyptusöl angeführt ist.

Darüber hinaus betrifft die US 2004/081699 A1 eine mucoadhäsive medizinische Zusammensetzung zur Verabreichung aktiver Substanzen in der Veterinär- oder Humanmedizin, wobei die Zusammensetzung mindestens eine aktive Substanz aufweisen soll. Die Zusammensetzung weist zudem eine mucoadhäsive Matrix auf, welche in einem wässrigen Medium löslich ist, wobei die Matrix ein matrixbildendes Polymer aufweist und wobei die aktive Substanz gelöst oder dispergiert vorliegt. Die Zusammensetzung soll maximal 15 Minuten nach Zugabe in ein wässriges Medium oder in Körperflüssigkeiten desintegrieren bzw. zerfallen.

Schließlich betrifft die auf die Anmelderin selbst zurückgehende DE 20 2012 002 792 U1 eine pharmazeutische Zusammensetzung insbesondere zur Behandlung von Rhinitiden, wobei die Zusammensetzung neben Pantothenol bzw. Pantothensäure mindestens ein imidazolinbasiertes alpha-Sympathomimetikum enthält.

Darüber hinaus sind aus dem Stand der Technik für die Behandlung von Rhinitiden auch sympathomimetikafreie, für die intranasale Applikation bestimmte Zusammensetzungen bekannt, welche aber oftmals keine ausreichende Wirksamkeit in Bezug auf eine effiziente Behandlung von Rhinitiden aufweisen.

Das der vorliegenden Erfindung zugrundeliegende Problem besteht daher in der Bereitstellung einer für die topische, insbesondere nasale, bevorzugt intranasale Applikation, insbesondere für die Behandlung von Rhinitiden, geeigneten Zusammensetzung, insbesondere pharmazeutischen Zusammensetzung, welche die zuvor geschilderten Nachteile des Standes der Technik zumindest weitgehend vermeidet oder aber wenigstens abschwächt.

Insbesondere soll eine solche Zusammensetzung eine gegenüber herkömmlichen, für die Behandlung von Rhinitiden mittels topischer bzw. intranasaler Applikation bestimmten, sympathomimetikahaltigen pharmazeutischen Präparaten verbesserte Wirkeffizienz und/oder ein verbessertes Nebenwirkungsprofil aufweisen.

Zur Lösung des zuvor geschilderten Problems schlägt die vorliegende Erfindung - gemäß einem ersten Aspekt der vorliegenden Erfindung - eine cineolhaltige Zusammensetzung, insbesondere eine pharmazeutische Zusammensetzung, nach Anspruch 1 vor; weitere, insbesondere vorteilhafte Ausgestaltungen der erfindungsgemäßen Zusammensetzung sind Gegenstand der diesbezüglichen Unteransprüche.

Weiterhin betrifft die vorliegende Erfindung - gemäß einem zweiten Aspekt der vorliegenden Erfindung - eine die erfindungsgemäße cineolhaltige Zusammensetzung enthaltende Applikationsvorrichtung gemäß dem betreffenden Vorrichtungsanspruch.

Es versteht sich bei den nachfolgenden Ausführungen von selbst, dass Ausgestaltungen, Ausführungsformen, Vorteile und dergleichen, welche nachfolgend zu Zwecken der Vermeidung von Wiederholungen nur zu einem Erfindungsaspekt ausgeführt sind, selbstverständlich auch in Bezug auf die übrigen Erfindungsaspekte entsprechend gelten, ohne dass dies einer gesonderten Erwähnung bedarf.

Bei allen nachstehend genannten relativen bzw. prozentualen gewichtsbezogenen Angaben, insbesondere Mengenangaben, ist weiterhin zu beachten, dass diese im Rahmen der vorliegenden Erfindung vom Fachmann derart auszuwählen sind, dass sie sich in der Summe unter Einbeziehung aller Komponenten bzw. Inhaltsstoffe, insbesondere wie nachfolgend definiert, stets zu 100 % bzw. 100 Gew.-% ergänzen bzw. addieren; dies versteht sich aber für den Fachmann von selbst.

Zudem gilt, dass alle im Folgenden genannten Werte- bzw. Parameterangaben oder dergleichen grundsätzlich mit genormten bzw. standardisierten oder explizit angegebenen Bestimmungsverfahren oder andernfalls mit dem Fachmann auf diesem Gebiet an sich geläufigen Bestimmungs- bzw. Messmethoden ermittelt bzw. bestimmt werden können.

Dies vorausgeschickt, wird die vorliegende Erfindung nunmehr nachfolgend im Detail erläutert.

Gegenstand der vorliegenden Erfindung - gemäß einem **ersten** Aspekt der vorliegenden Erfindung - ist somit eine cineolhaltige Zusammensetzung,
wobei die Zusammensetzung in Kombination und jeweils in wirksamen, insbesondere pharmazeutisch wirksamen Mengen enthält:
(a) 1,8-Cineol, wobei das Cineol als Reinstoff vorliegt, wobei das Cineol eine Reinheit von mindestens 95 Gew.-%, bezogen auf das Cineol, aufweist und wobei das Cineol frei von anderen Terpenen ist und wobei die Zusammensetzung die Komponente (a), bezogen auf die Zusammensetzung, in relativen Mengen im Bereich von 0,001 bis 10 Gew.-% enthält;
   und
(b)
   (b1) Pantothenol, vorzugsweise Dexpanthenol (D-Pantothenol), oder dessen physiologisch unbedenkliche Ester und/oder
   (b2) Pantothensäure oder deren physiologisch unbedenkliche Salze, wobei die Zusammensetzung die Komponente (b), bezogen auf die Zusammensetzung, in relativen Mengen im Bereich von 0,01 bis 10 Gew.-% enthält;
wobei die Zusammensetzung einen pH-Wert im Bereich von 5,0 bis 6,5 aufweist.

Die vorliegende Erfindung ist mit zahlreichen Vorteilen und Besonderheiten verbunden, welche nachfolgend in nicht beschränkender Weise diskutiert sind und als Indiz für Patentfähigkeit zu werten sind.

Im Rahmen der vorliegenden Erfindung wird ein effizient wirkendes Antirhinitismittel (Antirhinitikum) bereitgestellt, welches ohne die zwingende Anwesenheit von alpha-Sympathomimetika auskommt, so dass die unerwünschten Nebenwirkungen von alpha-Sympathomimetika vermieden werden können.

Die erfindungsgemäße Zusammensetzung besitzt infolge der Anwesenheit der Komponente (a), d. h. des Cineols, insbesondere in Form von 1,8-Cineol, bei deren bestimmungsgemäßer Verwendung nicht nur eine antibakterielle (d. h. bakterizide bzw. bakteriostatische), sondern darüber hinaus - wie die Anmelderin vollkommen überraschend gefunden hat - auch eine antivirale (d. h. viruzide bzw. virustatische) Wirkung.

Weiterhin wirkt die erfindungsgemäße Zusammensetzung entzündungshemmend bzw. antiinflammatorisch und zudem sekretolytisch bzw. schleimlösend. Auf diese Weise kann die erfindungsgemäße Zusammensetzung Entzündungen der Nasenschleimhaut (wie sie bei Rhinitiden stets auftreten) in effizienter Weise entgegenwirken. Zudem bewirkt die erfindungsgemäße Zusammensetzung eine gute Befeuchtung der Nasenschleimhaut.

Infolge des synergistischen Zusammenwirkens der Komponenten (a) und (b) wird insgesamt eine antirhinitische Wirkung erzielt. Insbesondere wird den Symptomen von Rhinitiden effizient entgegengewirkt, insbesondere auch dem sogenannten Fließschnupfen (Rhinorrhö) (d. h. die erfindungsgemäße Zusammensetzung bzw. Kombination besitzt auch ein antirhinorrhöisches Wirkprofil bzw. Wirkpotential).

Auch wirkt die erfindungsgemäße Zusammensetzung in effizienter Weise der bei Rhinitiden auftretenden Nasenatmungsbehinderung entgegen.

Die erfindungsgemäße Zusammensetzung wirkt aber nicht nur entzündungshemmend bzw. antiinflammatorisch, sondern besitzt insbesondere infolge der Anwesenheit der Komponente (b) auch schleimhautprotektive und wundheilungsfördernde Wirkung, so dass Entzündungen der Nasenschleimhaut, wie sie bei Rhinitiden stets auftreten, effizient entgegengewirkt wirkt. Zudem wirkt die Komponente (b) dahingehend, dass sie die Nasenschleimhaut pflegt und befeuchtet, so dass auf diese Weise auch der Austrocknung der Nasenschleimhaut und einer Borkenbildung entgegengewirkt wird.

Insbesondere aufgrund des antimikrobiellen, insbesondere bakteriziden bzw. bakteriostatischen Wirkpotentials des Cineols wird auch eine gute Stabilität, insbesondere Lagerungs- und Langzeitstabilität, der erfindungsgemäßen Zusammensetzung erreicht. Durch zusätzliche Maßnahmen (z. B. pH-Werteinstellung bzw. pH-Wertkontrolle sowie Verwendung chemischer Puffersysteme, Emulgatoren, Verdickungsmittel etc.) kann die Stabilität der erfindungsgemäßen Zusammensetzung weitergehend verbessert werden.

Durch optionale Inkorporierung weiterer Wirk- und/oder Inhaltsstoffe lassen sich Wirkprofil und Stabilitätsverhalten der erfindungsgemäßen Zusammensetzung kontrollieren bzw. gezielt einstellen.

Wie zuvor ausgeführt, enthält die cineolhaltige Zusammensetzung nach der vorliegenden Erfindung als Komponente (a) 1,8-Cineol.

Was den Wirkstoff 1,8-Cineol anbelangt, so ist hierzu Folgendes auszuführen: 1,8-Cineol gehört zu den bicyclischen Epoxy-Monoterpenen, genauer gesagt den Limonenoxiden. Synonyme Bezeichnungen für 1,8-Cineol mit der chemischen Summenformel C₁₀H₁₈O sind Cineol, Eucalyptol, Limonen-1,8-oxid, 1,8-Epoxy-p-menthan oder 1,3,3-Trimethyl-2-oxabicyclo[2.2.2]octan. Es handelt sich um eine farblose Flüssigkeit mit würzigem, campherähnlichem Geruch mit einem Schmelzpunkt von +1,5 °C und einem Siedepunkt von 176 bis 177 °C, welche in Wasser unlöslich, aber mit den meisten organischen Lösemitteln mischbar ist. Natürlich kommt 1,8-Cineol als Hauptbestandteil des Eukalyptusöls (Eukalyptusöl enthält bis zu 85 Gew.-% 1,8-Cineol), aber auch in anderen Pflanzen vor, so z. B. in Minze, Heilsalbei, Thymian, Basilikum und im Teebaum; darüber hinaus ist 1,8-Cineol beispielsweise in Niaouli-, Juniperus-, Piper-, Cannabis-, Kajeput-, Salbeiöl, Myrtenöl und anderen ätherischen Ölen enthalten. Technisches 1,8-Cineol, welches im Allgemeinen 99,6%-ig bis 99,8%-ig ist, wird im Allgemeinen durch fraktionierte Destillation von Eukalyptusöl gewonnen.

Für weitergehende Einzelheiten zu dem Wirkstoff 1,8-Cineol kann beispielsweise verwiesen werden auf RÖMPP Chemielexikon, Georg Thieme Verlag, Stuttgart/New York, 10. Auflage, Band 1, 1996, Seite 752, Stichwort: "Cineol", sowie auf die dort referierte Literatur.

Wie zuvor bereits ausgeführt, entfaltet das Cineol, insbesondere 1,8-Cineol, in der erfindungsgemäßen Zusammensetzung bei deren bestimmungsgemäßer Verwendung nicht nur eine antibakterielle (d. h. bakterizide bzw. bakteriostatische), sondern darüber hinaus (wie die Anmelderin vollkommen überraschend gefunden hat) auch eine antivirale (d. h. viruzide bzw. virustatische) Wirkung. Weiterhin wirkt das Cineol, insbesondere 1,8-Cineol, im Rahmen der erfindungsgemäßen Zusammensetzung insgesamt entzündungshemmend bzw. antiinflammatorisch und zudem sekretolytisch bzw. schleimlösend; auf diese Weise wirkt es - in synergistischer Kombination mit der Komponente (b) (d. h. Pantothenol bzw. Pantothensäure)-Entzündungen der Nasenschleimhaut effizient entgegen und sorgt zudem für eine gute Befeuchtung der Nasenschleimhaut. In synergistischer Zusammenwirkung mit der Komponente (b) wird insgesamt eine antirhinitische Wirkung erzielt und zudem den Symptomen von Rhinitiden effizient entgegengewirkt, insbesondere auch dem sogenannten Fließschnupfen (Rhinorrhö) (d. h. die erfindungsgemäße Zusammensetzung bzw. Kombination besitzt auch ein antirhinorrhöisches Wirkprofil bzw. Wirkpotential). Auch wirkt das Cineol in Zusammenwirken mit der Komponente (b) in effizienter Weise der bei Rhinitiden auftretenden Nasenatmungsbehinderung entgegen. Aufgrund seines antimikrobiellen, insbesondere bakteriziden bzw. bakteriostatischen Wirkpotentials ist das Cineol auch in Bezug auf die Stabilität, insbesondere Lagerungs- und Langzeitstabilität, der erfindungsgemäßen Zusammensetzung förderlich.

Erfindungsgemäß ist es vorgesehen, dass die Komponente (a) das Cineol als Reinstoff frei von anderen Terpenen enthält, vorzugsweise mit einer Reinheit von mindestens 96 Gew.-%, bevorzugt mindestens 97 Gew.-%, besonders bevorzugt mindestens 98 Gew.-%, ganz besonders bevorzugt mindestens 99 Gew.-%, noch mehr bevorzugt mindestens 99,5 %, bezogen auf das Cineol. Dies bedeutet, dass die Zusammensetzung außer dem Cineol kein weiteres Terpen enthält.

Mit anderen Worten liegt in der cineolhaltigen Zusammensetzung nach der vorliegenden Erfindung das Cineol als Reinstoff vor, d. h. das in der cineolhaltigen Zusammensetzung nach der vorliegenden Erfindung eingesetzte Cineol ist frei von anderen Terpenen bzw. enthält keine anderen Terpene.

In bevorzugter Weise weist das als Komponente (a) verwendete Cineol eine Reinheit von mindestens 95 Gew.-%, insbesondere mindestens 96 Gew.-%, bevorzugt mindestens 97 Gew.-%, besonders bevorzugt mindestens 98 Gew.-%, ganz besonders bevorzugt mindestens 99 Gew.-%, noch mehr bevorzugt mindestens 99,5 %, bezogen auf das Cineol, auf.

Gemäß einer besonderen Ausführungsform der vorliegenden Erfindung enthält die erfindungsgemäße Zusammensetzung das Cineol als 1,8-Cineol bzw. liegt das Cineol als 1,8-Cineol vor (d. h. mit anderen Worten enthält gemäß dieser besonderen Ausführungsform die Komponente (a) das Cineol in Form von 1,8-Cineol).

Durch die Verwendung von Cineol als Reinsubstanz, insbesondere in Form von 1,8-Cineol, wird ein definiertes oder steuerbares Wirkprofil gewährleistet. Insbesondere werden infolge der Abwesenheit weiterer Terpene unerwünschte oder unvorhersehbare Wechsel- und Nebenwirkungen vermieden.

Gemäß einer besonderen Ausführungsform der vorliegenden Erfindung kann die Komponente (a) bzw. das Cineol in liposomenumgebener und/oder liposomal verpackter Form vorliegen bzw. formuliert werden. Auf diese Weise wird insbesondere eine verbesserte Löslichkeit bzw. Emulgierbarkeit des Cineols bei gleichzeitig guter Stabilisierung des Cineols in der Zusammensetzung und bei guter Bioverfügbarkeit erreicht.

Die Menge an Komponente (a) bzw. Cineol in der cineolhaltigen Zusammensetzung nach der vorliegenden Erfindung kann in weiten Bereichen variieren bzw. eingestellt werden. Erfindungsgemäß ist es vorgesehen, dass die Zusammensetzung die Komponente (a) bzw. das Cineol, bezogen auf die Zusammensetzung, in relativen Mengen im Bereich von 0,001 bis 10 Gew.-%, insbesondere 0,002 bis 5 Gew.-%, vorzugsweise 0,01 bis 3 Gew.-%, bevorzugt 0,05 bis 3 Gew.-%, besonders bevorzugt 0,1 bis 2,5 Gew.-%, ganz besonders bevorzugt 0,2 bis 2 Gew.-%, noch mehr bevorzugt 0,3 bis 1 Gew.-%, am meisten bevorzugt 0,4 bis 0,8 Gew.-%, enthält.

Im Zusammenhang mit der Applikation bzw. Verabreichung der Komponente (a) (d. h. Cineol, vorzugsweise in Form von 1,8-Cineol) ist es erfindungsgemäß bevorzugt, wenn die Komponente (a) mit einer Einzeldosis im Bereich von 0,01 mg bis 50 mg, insbesondere im Bereich von 0,1 mg bis 25 mg, vorzugsweise im Bereich von 0,5 mg bis 20 mg, bevorzugt im Bereich von 1 mg bis 15 mg, besonders bevorzugt im Bereich von 2 mg bis 10 mg, verabreicht wird bzw. wenn die Komponente (a) zur Verabreichung mit einer Einzeldosis im Bereich von 0,01 mg bis 50 mg, insbesondere im Bereich von 0,1 mg bis 25 mg, vorzugsweise im Bereich von 0,5 mg bis 20 mg, bevorzugt im Bereich von 1 mg bis 15 mg, besonders bevorzugt im Bereich von 2 mg bis 10 mg, hergerichtet ist. In Bezug auf die zuvor genannten Einzeldosen ist es erfindungsgemäß zudem üblich, dass 2 bis 10 Einzeldosen pro Tag, insbesondere 3 bis 5 Einzeldosen pro Tag, verabreicht werden, vorzugsweise über den Tag verteilt, bzw. dass die Komponente (a) zur Verabreichung in 2 bis 10 Einzeldosen pro Tag, insbesondere 3 bis 5 Einzeldosen pro Tag, hergerichtet ist, vorzugsweise über den Tag verteilt.

Was die Komponente (b) der erfindungsgemäßen Zusammensetzung anbelangt, so kann diese Komponente (b) - wie zuvor bereits dargelegt - (b1) Pantothenol, vorzugsweise Dexpanthenol (D-Pantothenol), oder dessen physiologisch unbedenkliche Ester und/oder (b2) Pantothensäure oder deren physiologisch unbedenkliche Salze (Pantothenate) umfassen.

Wie zuvor ausgeführt, enthält also die cineolhaltige Zusammensetzung nach der vorliegenden Erfindung als Komponente (b) Pantothenol, vorzugsweise Dexpanthenol (D-Pantothenol), bzw. dessen physiologisch unbedenkliche Ester und/oder Pantothensäure bzw. deren physiologisch unbedenkliche Salze. Der Wirkstoff Pantothenol gehört in chemischer Hinsicht zu den Polyolen und Amiden und ist in physiologischer Hinsicht ein Provitamin (d. h. ein Präkursor eines Vitamins der Vitamin B-Gruppe), welches im Körper zu Pantothensäure (Vitamin B5) umgewandelt wird. Der Wirkstoff Pantothensäure wiederum ist ein Bestandteil des Coenzyms A und spielt damit eine wesentliche Rolle im (Schleim-)Hautstoffwechsel.

Wie zuvor bereits ausgeführt, entfaltet die Komponente (b) (d. h. Pantothenol, vorzugsweise Dexpanthenol, oder dessen physiologisch unbedenkliche Ester bzw. Pantothensäure oder deren physiologisch unbedenkliche Salze) in der erfindungsgemäßen Zusammensetzung bei deren bestimmungsgemäßer Verwendung nicht nur eine schleimhautprotektive und wundheilungsfördernde Wirkung, sondern wirkt auch eigenständig entzündungshemmend bzw. antiinflammatorisch. In synergistischer Kombination mit dem Cineol, insbesondere 1,8-Cineol, wird folglich Entzündungen der Nasenschleimhaut, wie sie bei Rhinitiden stets auftreten, effizient entgegengewirkt. Zudem wirkt die Komponente (b) die Nasenschleimhaut pflegend und befeuchtend, so dass auf diese Weise eine gute Befeuchtung der Nasenschleimhaut bewirkt wird bzw. einer Austrocknung der Nasenschleimhaut und einer Borkenbildung entgegengewirkt wird. Auch wird durch die Komponente (b) in Zusammenwirkung mit der Komponente (a) eine verbesserte Nasenatmung erreicht. In synergistischer Zusammenwirkung mit der Komponente (a) wird folglich insgesamt eine antirhinitische Wirkung erzielt und zudem den Symptomen von Rhinitiden effizient entgegengewirkt, insbesondere auch dem Fließschnupfen (Rhinorrhö) (d. h. die erfindungsgemäße Zusammensetzung bzw. Kombination besitzt - wie zuvor angeführt - auch ein antirhinorrhöisches Wirkprofil bzw. Wirkpotential).

Erfindungsgemäß bevorzugt ist es, wenn die Zusammensetzung als Komponente (b) Pantothenol, vorzugsweise Dexpanthenol (D-Pantothenol), oder dessen physiologisch unbedenkliche Ester enthält.

Erfindungsgemäß besonders bevorzugt ist es, wenn die Zusammensetzung als Komponente (b) Pantothenol, bevorzugt in Form von Dexpanthenol (D-Pantothenol), enthält.

Die Menge an Komponente (b) in der cineolhaltigen Zusammensetzung nach der vorliegenden Erfindung kann in weiten Bereichen variieren bzw. eingestellt werden. Erfindungsgemäß ist es vorgesehen, dass die erfindungsgemäße Zusammensetzung die Komponente (b), bezogen auf die Zusammensetzung, in relativen Mengen im Bereich von 0,01 bis 10 Gew.-%, insbesondere 0,1 bis 8 Gew.-%, vorzugsweise 0,5 bis 7 Gew.-%, bevorzugt 1 bis 6,5 Gew.-%, besonders bevorzugt 2 bis 6 Gew.-%, ganz besonders bevorzugt 4 bis 6 Gew.-%, noch mehr bevorzugt 4,5 bis 5,5 Gew.-%, enthält.

Im Zusammenhang mit der Applikation bzw. Verabreichung der Komponente (b) (d. h. Pantothenol bzw. Pantothensäure) ist es erfindungsgemäß bevorzugt, wenn die Komponente (b), vorzugsweise in Form von Pantothenol, besonders bevorzugt Dexpanthenol, mit einer Einzeldosis im Bereich von 0,1 mg bis 50 mg, insbesondere im Bereich von 0,5 mg bis 25 mg, vorzugsweise im Bereich von 1 mg bis 20 mg, bevorzugt im Bereich von 2,5 mg bis 10 mg, besonders bevorzugt im Bereich von 4 mg bis 8 mg, verabreicht wird bzw. wenn die Komponente (b), vorzugsweise in Form von Pantothenol, besonders bevorzugt Dexpanthenol, zur Verabreichung mit einer Einzeldosis im Bereich von 0,1 mg bis 50 mg, insbesondere im Bereich von 0,5 mg bis 25 mg, vorzugsweise im Bereich von 1 mg bis 20 mg, bevorzugt im Bereich von 2,5 mg bis 10 mg, besonders bevorzugt im Bereich von 4 mg bis 8 mg, hergerichtet ist. In Bezug auf die zuvor genannten Einzeldosen ist es erfindungsgemäß zudem üblich, dass 2 bis 10 Einzeldosen pro Tag, insbesondere 3 bis 5 Einzeldosen pro Tag, verabreicht werden, vorzugsweise über den Tag verteilt, bzw. dass die Komponente (b), vorzugsweise in Form von Pantothenol, besonders bevorzugt Dexpanthenol, zur Verabreichung in 2 bis 10 Einzeldosen pro Tag, insbesondere 3 bis 5 Einzeldosen pro Tag, hergerichtet ist, vorzugsweise über den Tag verteilt.

Für die Wirksamkeit der Komponenten (a) und (b) in der erfindungsgemäßen Zusammensetzung ist auch deren Mengenverhältnis von Bedeutung. Gemäß einer erfindungsgemäß bevorzugten Ausführungsform kann es vorgesehen sein, dass die erfindungsgemäße Zusammensetzung die Komponenten (a) und (b) in einem Mengenverhältnis von Komponente (a) zu Komponente (b) im Bereich von 1 : 1 bis 1 : 500, insbesondere 1 : 1,25 bis 1 : 100, vorzugsweise 1 : 1,5 bis 1 : 75, bevorzugt 1 : 2 bis 1 : 50, besonders bevorzugt 1 : 2,5 bis 1 : 25, ganz besonders bevorzugt 1 : 2,75 bis 1 : 20, noch mehr bevorzugt 1 : 3 bis 1 : 10, enthält. Dennoch kann es einzelfallbedingt oder anwendungsbezogen erforderlich sein, von den vorgenannten Werten abzuweichen, ohne dass der Rahmen der vorliegenden Erfindung verlassen ist (was im Ermessen des Fachmanns liegt).

Gemäß einer besonderen Ausführungsform der vorliegenden Erfindung kann es vorgesehen sein, dass die Zusammensetzung nach der vorliegenden Erfindung außerdem als Komponente (c) mindestens einen Emulgator enthält.

Ein Emulgator im Sinne der vorliegenden Erfindung ist ein Formulierungshilfsstoff, welcher dazu dient, zwei miteinander nicht oder nur geringfügig mischbare Stoffe bzw. Flüssigkeiten zu einem fein verteilten Gemisch (d. h. Emulsion) zu vermengen und zu stabilisieren. Im Rahmen der vorliegenden Erfindung soll insbesondere die Mischbarkeit bzw. Löslichkeit der Komponente (a), d. h. des Cineols, aber gegebenenfalls auch der Komponente (b), mit bzw. in dem wässrigen Exzipienten (Träger) der erfindungsgemäßen Zusammensetzung verbessert und die resultierende Emulsion stabilisiert werden. Auf diese Weise werden aber nicht nur die Mischbarkeit bzw. Emulgierbarkeit der Wirkstoffe in dem wasserbasierten Exzipienten verbessert und die Stabilität der resultierenden erfindungsgemäßen Zusammensetzung erhöht, sondern wird auch die Bioverfügbarkeit der Wirkstoffe, insbesondere des Cineols, verbessert.

Grundsätzlich ist die Verwendung eines Emulgators in Bezug auf die erfindungsgemäße Zusammensetzung zwar optional, stellt aber aus den vorgenannten Gründen eine bevorzugte Ausführungsform dar.

Die Menge an Komponente (c) bzw. Emulgator in der cineolhaltigen Zusammensetzung nach der vorliegenden Erfindung kann in weiten Bereichen variieren bzw. eingestellt werden. Insbesondere kann es vorgesehen sein, dass die erfindungsgemäße Zusammensetzung die Komponente (c) und/oder den Emulgator, bezogen auf die Zusammensetzung, in relativen Mengen im Bereich von 0,0001 bis 10 Gew.-%, insbesondere 0,001 bis 5 Gew.-%, vorzugsweise 0,005 bis 4 Gew.-%, bevorzugt 0,01 bis 3 Gew.-%, besonders bevorzugt 0,05 bis 2,5 Gew.-%, ganz besonders bevorzugt 0,1 bis 2 Gew.-%, noch mehr bevorzugt 0,2 bis 1,8 Gew.-%, am meisten bevorzugt 0,5 bis 1,5 Gew.-%, enthält.

Dennoch kann es einzelfallbedingt oder anwendungsbezogen erforderlich sein, von den vorgenannten Werten abzuweichen, ohne dass der Rahmen der vorliegenden Erfindung verlassen ist. Dies liegt im Ermessen des Fachmanns.

Die Komponente (c) bzw. der Emulgator kann grundsätzlich aus einer Vielzahl unterschiedlichster Verbindungen ausgewählt werden. Gemäß einer bevorzugten Ausführungsform kann es vorgesehen sein, dass die Komponente (c) bzw. der Emulgator ausgewählt ist aus der Gruppe von (i) Fettsäuren, Fettsäuresalzen, Fettsäureamiden und Fettsäureestern, insbesondere Fettsäureestern, (ii) gegebenenfalls (poly)ethoxylierten und/oder hydrierten (d. h. partiell oder vollständig hydrierten) Rizinusölen (= Castorölen), (iii) organischen Carbonsäuren und deren Salzen und Estern, insbesondere Citronensäure und deren Salzen (Citraten), (iv) organischen Betainen, (v) organischen Ammoniumverbindungen, (vi) physiologisch unbedenklichen ionischen oder nichtionischen Tensiden, (vii) Phospholipiden, insbesondere Phosphatidylcholinen und Lecithinen, (viii) Poloxameren (Ethylenoxid/Propylenoxid-Blockcopolymeren), (ix) Estern von mehrwertigen Alkoholen (Polyalkoholen), insbesondere Glycerinestern, (x) Glykolen, insbesondere Polyalkylenglykolen, vorzugsweise Polyethylenglykolen, und deren (Poly-)Ethern und (Poly-)Estern, sowie Kombinationen aus zwei oder mehreren der vorgenannten Verbindungen.

In besonders bevorzugter Weise kann die Komponente (c) bzw. der Emulgator ausgewählt sein aus der Gruppe von (i) Fettsäuren, Fettsäuresalzen, Fettsäureamiden und Fettsäureestern, insbesondere Fettsäureestern, und (ii) gegebenenfalls (poly)ethoxylierten und/oder hydrierten Rizinusölen sowie deren Kombinationen.

Des Weiteren kann es vorgesehen sein, dass die Zusammensetzung nach der Erfindung außerdem als Komponente (d) mindestens ein chemisches Puffersystem, insbesondere in Form von Puffersalz(en), enthält.

Zum Begriff des chemischen Puffers bzw. des chemischen Puffersystems kann insbesondere verwiesen werden auf RÖMPP Lexikon Chemie, 10. Auflage, Georg-Thieme-Verlag, Stuttgart/New York, Band 5, 1998, Seiten 3618/3619, Stichwort: *"Puffer",* sowie auf die dort referierte Literatur.

Das chemische Puffersystem, insbesondere in Form von Puffersalz(en), dient im Rahmen der vorliegenden Erfindung insbesondere zur Stabilisierung der erfindungsgemäßen Zusammensetzung. Insbesondere führt die Einstellung eines konstanten pH-Werts mittels des Puffersystems überraschenderweise dazu, dass einem unerwünschten Abbau der Wirkstoffe, insbesondere der Komponenten (a) und (b), bei Lagerung auch über einen längeren Zeitraum effizient entgegengewirkt wird. Auf diese Weise wird die Lagerfähigkeit der erfindungsgemäßen Zusammensetzung verbessert.

Die Menge an Komponente (d) bzw. chemischem Puffersystem in der cineolhaltigen Zusammensetzung nach der vorliegenden Erfindung kann in weiten Bereichen variieren bzw. eingestellt werden. Erfindungsgemäß bevorzugt ist es, wenn die erfindungsgemäße Zusammensetzung die Komponente (d) bzw. das chemische Puffersystem, bezogen auf die Zusammensetzung und berechnet als Summe aller Bestandteile des chemischen Puffersystems, in relativen Mengen im Bereich von 0,001 bis 4 Gew.-%, insbesondere 0,01 bis 3 Gew.-%, vorzugsweise 0,05 bis 2 Gew.-%, bevorzugt 0,1 bis 1,5 Gew.-%, besonders bevorzugt 0,2 bis 1 Gew.-%, enthält.

Im Rahmen der vorliegenden Erfindung dient die Komponente (d) bzw. das chemische Puffersystem insbesondere zur Einstellung und/oder Konstanthaltung des pH-Werts der erfindungsgemäßen Zusammensetzung. Wie zuvor ausgeführt, führt dies dazu, dass einem unerwünschten Abbau der Wirkstoffe, insbesondere der Komponenten (a) und (b), bei Lagerung auch über einen längeren Zeitraum effizient entgegengewirkt und auf diese Weise die Lagerfähigkeit der erfindungsgemäßen Zusammensetzung verbessert wird.

In erfindungsgemäß bevorzugter Weise kann es vorgesehen sein, dass die Komponente (d) bzw. das chemische Puffersystem als ein Dihydrogenphosphat/Monohydrogenphosphat-Puffersystem (synonym bisweilen auch als "H₂PO₄⁻/HPO₄²⁻-Puffer(system)" bzw. "Phosphatpuffer(system)" bezeichnet), insbesondere als ein Alkalidihydrogenphosphat/Alkalimonohydrogenphosphat-Puffersystem, vorliegt, bevorzugt mit einem Dihydrogenphosphat/Monohydrogenphosphat-Molverhältnis von größer 5 : 1, insbesondere im Bereich von 5 : 1 bis 110 : 1, besonders bevorzugt im Bereich von 6 : 1 bis 105 : 1, ganz besonders bevorzugt im Bereich von 7 : 1 bis 200 : 1, noch mehr bevorzugt im Bereich von 7 : 1 bis 100 : 1. Bei Verwendung dieses speziellen Puffersystems, insbesondere bei gleichzeitiger Einhaltung der vorgenannten Molverhältnisse, wird einem unerwünschten Abbau der Wirkstoffe, insbesondere der Komponenten (a) und (b), bei Lagerung auch über einen längeren Zeitraum in besonderem Maße entgegengewirkt und wird die Lagerfähigkeit der erfindungsgemäßen Zusammensetzung in besonderem Maße verbessert.

Grundsätzlich kann der pH-Wert der Zusammensetzung in weiten Bereichen variieren. Erfindungsgemäß ist es vorgesehen, dass die erfindungsgemäße Zusammensetzung einen pH-Wert im Bereich von 5,0 bis 6,5, vorzugsweise im Bereich von 5,0 bis 6,2, bevorzugt im Bereich von 5,0 bis 6,0, noch mehr bevorzugt im Bereich von 5,1 bis 6,0, ganz besonders bevorzugt im Bereich von 5,2 bis 5,9, am meisten bevorzugt im Bereich von 5,3 bis 5,9, aufweist bzw. wenn der pH-Wert der erfindungsgemäßen Zusammensetzung im Bereich von 4,5 bis 8,0, insbesondere im Bereich von 5,0 bis 6,5, vorzugsweise im Bereich von 5,0 bis 6,2, bevorzugt im Bereich von 5,0 bis 6,0, noch mehr bevorzugt im Bereich von 5,1 bis 6,0, ganz besonders bevorzugt im Bereich von 5,2 bis 5,9, am meisten bevorzugt im Bereich von 5,3 bis 5,9, eingestellt und/oder konstantgehalten wird (vorzugsweise mittels mindestens eines chemischen Puffersystems, insbesondere wie zuvor definiert). Bei Einhaltung der vorgenannten pH-Wertebereiche wird einem unerwünschten Abbau der Wirkstoffe, insbesondere der Komponenten (a) und (b), bei Lagerung auch über einen längeren Zeitraum in besonderem Weise entgegengewirkt und wird auch die Lagerfähigkeit der erfindungsgemäßen Zusammensetzung in besonderem Maße verbessert.

Im Rahmen der vorliegenden Erfindung kann die Bestimmung des pH-Werts mit dem Fachmann an sich bekannten Methoden erfolgen. Insbesondere kann die Bestimmung des pH-Werts gemäß der Methode nach Ph. Eur. [Pharmacopoea Europaea] 7. Ausgabe, Grundwerk 2011, Abschnitt 2.2.3, erfolgen.

Gemäß einer besonderen Ausführungsform der vorliegenden Erfindung kann es vorgesehen sein, dass die erfindungsgemäße Zusammensetzung außerdem als Komponente (e) mindestens ein Konservierungsmittel und/oder Desinfektionsmittel (Antiseptikum) enthält.

Ein Konservierungsmittel und/oder Desinfektionsmittel (Antiseptikum) im Sinne der vorliegenden Erfindung ist ein Formulierungshilfsstoff, welcher dazu dient, Mikroorganismen entgegenzuwirken und/oder antimikrobielle bzw. wunddesinfizierende Wirkung zu entfalten. Im Rahmen der vorliegenden Erfindung soll durch die Verwendung der Komponente (e) einem (übermäßigen) Befall der erfindungsgemäßen Zusammensetzung mit Mikroorganismen und somit einer unerwünschten Verkeimung entgegengewirkt werden. Auf diese Weise wird die Stabilität der resultierenden erfindungsgemäßen Zusammensetzung, insbesondere auch im Hinblick auf eine Lagerung über längere Zeiträume, verbessert. Wie die Anmelderin überraschend gefunden hat, unterstützt die Anwesenheit eines Konservierungsmittels und/oder Desinfektionsmittels (Antiseptikums) aber auch das antiinflammatorische und antibakterielle wie antivirale Wirkprofil des Cineols im Rahmen der Applikation der erfindungsgemäßen Zusammensetzung bei der Behandlung von Rhinitiden.

Grundsätzlich ist die Verwendung eines Konservierungsmittels und/oder Desinfektionsmittels (Antiseptikums) in Bezug auf die erfindungsgemäße Zusammensetzung zwar optional, stellt aber aus den vorgenannten Gründen eine bevorzugte Ausführungsform dar.

Die Menge an Komponente (e) bzw. Konservierungsmittel und/oder Desinfektionsmittel (Antiseptikum) in der cineolhaltigen Zusammensetzung nach der vorliegenden Erfindung kann in weiten Bereichen variieren bzw. eingestellt werden. Insbesondere kann es vorgesehen sein, dass die erfindungsgemäße Zusammensetzung die Komponente (e) bzw. das Konservierungsmittel und/oder Desinfektionsmittel (Antiseptikum), bezogen auf die Zusammensetzung, in relativen Mengen im Bereich von 0,001 bis 10 Gew.-%, insbesondere 0,005 bis 5 Gew.-%, vorzugsweise 0,01 bis 2 Gew.-%, bevorzugt 0,01 bis 1 Gew.-%, besonders bevorzugt 0,01 bis 0,5 Gew.-%, noch mehr bevorzugt 0,02 bis 0,1 Gew.-%, enthält. Dennoch kann es einzelfallbedingt oder anwendungsbezogen erforderlich sein, von den vorgenannten Werten abzuweichen, ohne dass der Rahmen der vorliegenden Erfindung verlassen ist. Dies liegt im Ermessen des Fachmanns.

Die Komponente (e) bzw. das Konservierungsmittel und/oder Desinfektionsmittel (Antiseptikum) kann grundsätzlich aus einer Vielzahl unterschiedlichster Verbindungen ausgewählt werden. Gemäß einer bevorzugten Ausführungsform kann es vorgesehen sein, dass die Komponente (e) bzw. das Konservierungsmittel und/oder Desinfektionsmittel (Antiseptikum) ausgewählt ist aus der Gruppe von (i) Alkylbenzyldimethylammoniumchloriden, insbesondere C₈-C₁₈-Alkylbenzyldimethylammoniumchloriden, und Mischungen von verschiedenen Alkylbenzyldimethylammoniumchloriden, vorzugsweise Benzalkoniumchlorid, (ii) Polyhexanid (iii) Sorbinsäure und deren Salzen (Sorbaten), insbesondere Kaliumsorbat, (iv) Chlorhexidin, (v) para-Hydroxybenzoesäureestern und Mischungen von verschiedenen para-Hydroxybenzoesäureestern, vorzugsweise Nipa-Estern, sowie Kombinationen aus zwei oder mehreren der vorgenannten Verbindungen, bevorzugt aus der aus der Gruppe von (i) Alkylbenzyldimethylammoniumchloriden, insbesondere C₈-C₁₈-Alkylbenzyldimethylammoniumchloriden, und Mischungen von verschiedenen Alkylbenzyldimethylammoniumchloriden, vorzugsweise Benzalkoniumchlorid, (ii) Polyhexanid (iii) Sorbinsäure und deren Salzen (Sorbaten), insbesondere Kaliumsorbat, sowie Kombinationen aus zwei oder mehreren der vorgenannten Verbindungen. In besonders bevorzugter Weise ist die Komponente (e) bzw. das Konservierungsmittel und/oder Desinfektionsmittel (Antiseptikum) ausgewählt aus der Gruppe von Benzalkoniumchlorid, Polyhexanid und Sorbaten (z. B. Kaliumsorbat) sowie deren Kombinationen. Erfindungsgemäß als Komponente (e) ganz besonders bevorzugt ist Benzalkoniumchlorid (d. h. ein Gemisch von Alkylbenzyldimethylammoniumchloriden, deren Alkylteil aus C₈-C₁₈-Ketten besteht)

Weiterhin kann es vorgesehen sein, dass die erfindungsgemäße Zusammensetzung außerdem als Komponente (f) mindestens ein Verdickungsmittel enthält.

Ein Verdickungsmittel im Sinne der vorliegenden Erfindung ist ein Formulierungshilfsstoff, welcher dazu dient, die Viskosität der erfindungsgemäßen Zusammensetzung zu erhöhen (d. h. die Zusammensetzung zähflüssiger bzw. weniger fließfähig auszubilden). Im Rahmen der vorliegenden Erfindung soll durch die Verwendung der Komponente (f) erreicht werden, dass die erfindungsgemäße Zusammensetzung bei ihrer intranasalen Applikation länger an der Nasenschleimhaut haftet und auf diese Weise die Wirkdauer verlängert wird. Insbesondere wird auf diese Weise die sogenannte Bioadhäsion verbessert. Auch kann auf diese Weise die Wirkstofffreisetzung gezielt kontrolliert werden. Aufgrund der infolge der Anwesenheit des Verdickungsmittels verbesserten mucoadhäsiven Wirkung können eine längere Verweildauer am Wirkort und eine verbesserte Befeuchtung der Nasenschleimhaut erreicht werden.

Die Menge an Komponente (f) bzw. Verdickungsmittel in der cineolhaltigen Zusammensetzung nach der vorliegenden Erfindung kann in weiten Bereichen variieren bzw. eingestellt werden. Insbesondere kann es vorgesehen sein, dass die erfindungsgemäße Zusammensetzung die Komponente (f) bzw. das Verdickungsmittel, bezogen auf die Zusammensetzung, in relativen Mengen im Bereich von 0,0001 bis 10 Gew.-%, insbesondere 0,001 bis 8 Gew.-%, vorzugsweise 0,005 bis 5 Gew.-%, bevorzugt 0,01 bis 3 Gew.-%, besonders bevorzugt 0,05 bis 2 Gew.-%, noch mehr bevorzugt 0,1 bis 1 Gew.-%, enthält. Dennoch kann es einzelfallbedingt oder anwendungsbezogen erforderlich sein, von den vorgenannten Werten abzuweichen, ohne dass der Rahmen der vorliegenden Erfindung verlassen ist. Dies liegt im Ermessen des Fachmanns.

Die Komponente (f) bzw. das Verdickungsmittel kann grundsätzlich aus einer Vielzahl unterschiedlichster Verbindungen ausgewählt werden. Gemäß einer bevorzugten Ausführungsform kann es vorgesehen sein, dass die Komponente (f) bzw. das Verdickungsmittel ausgewählt ist aus der Gruppe von (i) vorzugsweise sauren Glykosaminoglykanen oder deren physiologisch unbedenkliche Salzen oder Derivaten, insbesondere Hyaluronsäuren oder deren physiologisch unbedenklichen Salzen (Hyaluronaten), (ii) Polysacchariden und Hydrokolloiden, insbesondere Carrageenen (Carrageenanen) (iii) Cellulosen und Cellulosederivaten, insbesondere Celluloseestern und Celluloseethern, (iv) Alginsäuren und deren Salzen (Alginaten), (v) Poly(meth)acrylsäuren und Poly(meth)acrylaten, (vi) Polyalkylenglykolen, insbesondere Polyethylenglykolen, (vii) Xanthanen sowie Kombinationen aus zwei oder mehreren der vorgenannten Verbindungen.

In besonders bevorzugter Weise ist die Komponente (f) bzw. das Verdickungsmittel ausgewählt aus der aus der Gruppe von Hyaluronsäuren oder deren physiologisch unbedenklichen Salze (Hyaluronaten), Carrageenen (Carrageenanen) sowie deren Kombinationen.

Für den Fall, dass als Komponente (f) bzw. als Verdickungsmittel eine Cellulose oder Cellulosederivat verwendet wird, kommt insbesondere Hydroxypropylmethylcellulose (synonym auch als "HPMC", "Hypromellose" etc. bezeichnet) oder deren Derivate (insbesondere Ether und/oder Ester) zum Einsatz. Hydroxypropylmethylcellulose (HPMC) ist ein Stoffgemisch aus verschiedenen, teilweise alkylsubstituierten Cellulosen. Sie ist in verschiedenen Polymerisationsgraden und unterschiedlichen Substitutionsgraden kommerziell verfügbar.

Weiterhin kann es vorgesehen sein, dass die erfindungsgemäße Zusammensetzung außerdem als Komponente (g) mindestens ein Ectoin oder Ectoinderivat, insbesondere ein Hydroxyectoin, enthält, insbesondere in relativen Mengen im Bereich von 0,0001 bis 10 Gew.-%, insbesondere 0,001 bis 5 Gew.-%, vorzugsweise 0,01 bis 2 Gew.-%, bezogen auf die Zusammensetzung.

Gleichermaßen kann es vorgesehen sein, dass die erfindungsgemäße Zusammensetzung außerdem als Komponente (h) mindestens ein vorzugsweise imidazolinbasiertes alpha-Sympathomimetikum oder dessen physiologisch unbedenkliche Salze enthält; dies ist insbesondere der Fall, wenn zusätzlich eine dekongestive bzw. abschwellende Wirkung der erfindungsgemäßen Zusammensetzung im besonderen Maße gewünscht ist (auch wenn die Anwesenheit eines alpha-Sympathomimetikums für den Therapieerfolg der erfindungsgemäßen Zusammensetzung grundsätzlich nicht erforderlich ist). Bei dieser Ausführungsform kann das vorzugsweise imidazolinbasierte alpha-Sympathomimetikum insbesondere ausgewählt sein aus Xylometazolin oder Oxymetazolin, insbesondere in Form von deren physiologisch unbedenklichen Salzen, besonders bevorzugt in Form von deren Hydrochloridsalzen, und besonders bevorzugt Xylometazolinhydrochlorid sein. Bei dieser Ausführungsform kann die erfindungsgemäße Zusammensetzung das vorzugsweise imidazolinbasierte alpha-Sympathomimetikum, bezogen auf die Zusammensetzung, in relativen Mengen im Bereich von 0,001 bis 2 Gew.-%, insbesondere 0,005 bis 1,5 Gew.-%, vorzugsweise 0,01 bis 1,2 Gew.-%, bevorzugt 0,02 bis 1,0 Gew.-%, besonders bevorzugt 0,03 bis 0,5 Gew.-%, ganz besonders bevorzugt 0,04 bis 0,2 Gew.-%, enthalten.

Darüber hinaus kann es vorgesehen sein, dass die erfindungsgemäße Zusammensetzung außerdem Natriumchlorid enthält, insbesondere in relativen Mengen im Bereich von 0,001 bis 5 Gew.-%, insbesondere 0,01 bis 2 Gew.-%, vorzugsweise 0,1 bis 1 Gew.-%, bezogen auf die Zusammensetzung. Dies kann insbesondere der Fall sein, wenn eine zusätzliche Befeuchtung der Nasenschleimhaut beabsichtigt ist.

Gemäß einer besonderen Ausführungsform betrifft die vorliegende Erfindung eine cineolhaltige Zusammensetzung, insbesondere eine pharmazeutische Zusammensetzung, welche vorzugsweise für die topische, insbesondere nasale, bevorzugt intranasale Applikation, insbesondere für die Behandlung von Rhinitiden, geeignet ist, insbesondere eine wie zuvor beschriebene Zusammensetzung nach der vorliegenden Erfindung,
wobei die Zusammensetzung in Kombination und jeweils in wirksamen, insbesondere pharmazeutisch wirksamen Mengen enthält:
(a) Cineol, vorzugsweise 1,8-Cineol, in relativen Mengen im Bereich von 0,001 bis 10 Gew.-%, insbesondere 0,002 bis 5 Gew.-%, vorzugsweise 0,01 bis 3 Gew.-%, bevorzugt 0,05 bis 3 Gew.-%, besonders bevorzugt 0,1 bis 2,5 Gew.-%, ganz besonders bevorzugt 0,2 bis 2 Gew.-%, noch mehr bevorzugt 0,3 bis 1 Gew.-%, am meisten bevorzugt 0,4 bis 0,8 Gew.-%;
(b) Pantothenol, vorzugsweise Dexpanthenol (D-Pantothenol), oder dessen physiologisch unbedenkliche Ester, in relativen Mengen im Bereich von 0,01 bis 10 Gew.-%, insbesondere 0,1 bis 8 Gew.-%, vorzugsweise 0,5 bis 7 Gew.-%, bevorzugt 1 bis 6,5 Gew.-%, besonders bevorzugt 2 bis 6 Gew.-%, ganz besonders bevorzugt 4 bis 6 Gew.-%, noch mehr bevorzugt 4,5 bis 5,5 Gew.-%;
(c) gegebenenfalls mindestens einen Emulgator, insbesondere in relativen Mengen im Bereich von 0,0001 bis 5 Gew.-%, insbesondere 0,001 bis 2,5 Gew.-%, vorzugsweise 0,005 bis 2 Gew.-%, bevorzugt 0,01 bis 1,5 Gew.-%, besonders bevorzugt 0,01 bis 1 Gew.-%, ganz besonders bevorzugt 0,01 bis 0,5 Gew.-%, noch mehr bevorzugt 0,02 bis 0,08 Gew.-%;
(d) gegebenenfalls mindestens ein chemisches Puffersystem, insbesondere in Form von Puffersalz(en), insbesondere in relativen Mengen, berechnet als Summe aller Bestandteile des chemischen Puffersystems, im Bereich von 0,001 bis 4 Gew.-%, insbesondere 0,01 bis 3 Gew.-%, vorzugsweise 0,05 bis 2 Gew.-%, bevorzugt 0,1 bis 1,5 Gew.-%, besonders bevorzugt 0,2 bis 1 Gew.-%;
(e) gegebenenfalls mindestens ein Konservierungsmittel und/oder Desinfektionsmittel (Antiseptikum), insbesondere in relativen Mengen im Bereich von 0,001 bis 10 Gew.-%, insbesondere 0,005 bis 5 Gew.-%, vorzugsweise 0,01 bis 2 Gew.-%, bevorzugt 0,01 bis 1 Gew.-%, besonders bevorzugt 0,01 bis 0,5 Gew.-%, noch mehr bevorzugt 0,02 bis 0,1 Gew.-%;
(f) gegebenenfalls mindestens ein Verdickungsmittel, insbesondere in relativen Mengen im Bereich von 0,0001 bis 10 Gew.-%, insbesondere 0,001 bis 8 Gew.-%, vorzugsweise 0,005 bis 5 Gew.-%, bevorzugt 0,01 bis 3 Gew.-%, besonders bevorzugt 0,05 bis 2 Gew.-%, noch mehr bevorzugt 0,1 bis 1 Gew.-%;
(g) gegebenenfalls mindestens ein Ectoin oder Ectoinderivat, insbesondere ein Hydroxyectoin, insbesondere in relativen Mengen im Bereich von 0,0001 bis 10 Gew.-%, insbesondere 0,001 bis 5 Gew.-%, vorzugsweise 0,01 bis 2 Gew.-%;
(h) gegebenenfalls mindestens ein vorzugsweise imidazolinbasiertes alpha-Sympathomimetikum oder dessen physiologisch unbedenkliche Salze, insbesondere in relativen Mengen im Bereich von 0,001 bis 2 Gew.-%, insbesondere 0,005 bis 1,5 Gew.-%, vorzugsweise 0,01 bis 1,2 Gew.-%, bevorzugt 0,02 bis 1,0 Gew.-%, besonders bevorzugt 0,03 bis 0,5 Gew.-%, ganz besonders bevorzugt 0,04 bis 0,2 Gew.-%
(i) Natriumchlorid, insbesondere in relativen Mengen im Bereich von 0,001 bis 5 Gew.-%, insbesondere 0,01 bis 2 Gew.-%, vorzugsweise 0,1 bis 1 Gew.-%;
wobei alle vorgenannten relativen Mengenangaben auf die Zusammensetzung bezogen sind.

Die cineolhaltige Zusammensetzung nach der vorliegenden Erfindung kann grundsätzlich in unterschiedlichsten Erscheinungsformen vorliegen und/oder in unterschiedlichsten Formulierungsformen ausgestaltet sein.

Im Allgemeinen kann die Zusammensetzung nach der vorliegenden Erfindung als wässrige Zusammensetzung vorliegen. Insbesondere kann die Zusammensetzung nach der vorliegenden Erfindung wässrig basiert sein und/oder wässrig formuliert vorliegen, insbesondere in Form einer wässrigen Lösung oder wässrigen Solubilisierung. Auf diese Weise werden eine gute physiologische Kompatibilität und eine gute Applizierbarkeit sichergestellt.

Gemäß einer bevorzugten Ausführungsform kann die erfindungsgemäße cineolhaltige Zusammensetzung als wässriges System, insbesondere als wässriges Einphasensystem, vorzugsweise als wässrige Lösung oder wässrige Solubilisierung, vorliegen. Im Allgemeinen weist die Zusammensetzung nach der vorliegenden Erfindung einen Exzipienten oder Träger auf Wasserbasis auf. Bevorzugterweise liegt die Zusammensetzung nach der Erfindung als klare, farblose wässrige Lösung vor. Dies gewährleistet einerseits eine gute physiologische Verträglichkeit sowie eine gute Anwendbarkeit und andererseits eine gute Stabilität, insbesondere Lagerstabilität, und zwar auch über lange Zeiträume.

Im Rahmen der vorliegenden Erfindung kann die Bestimmung des Aussehens der erfindungsgemäßen Zusammensetzung mit dem Fachmann an sich bekannten Methoden erfolgen. Insbesondere kann die Bestimmung des Aussehens der erfindungsgemäßen Zusammensetzung gemäß der Methode nach Ph. Eur. [Pharmacopoea Europaea] 7. Ausgabe, Grundwerk 2011, Abschnitte 2.2.1 und 2.2.2, erfolgen.

Auch die Osmolalität der cineolhaltigen Zusammensetzung nach der vorliegenden Erfindung kann in weiten Grenzen variieren. Zur Gewährleistung einer guten physiologischen Kompatibilität bei gleichzeitig guter Applizierbarkeit hat es sich bewährt, wenn die erfindungsgemäße Zusammensetzung eine Osmolalität im Bereich von 300 bis 600 mosm/kg, insbesondere im Bereich von 310 bis 550 mosm/kg, vorzugsweise im Bereich von 300 bis 525 mosm/kg, bevorzugt im Bereich von 325 bis 510 mosm/kg, besonders bevorzugt im Bereich von 350 bis 500 mosm/kg, aufweist. Dennoch kann es einzelfallbedingt oder anwendungsbezogen erforderlich sein, von den vorgenannten Werten abzuweichen, ohne dass der Rahmen der vorliegenden Erfindung verlassen ist; dies liegt im freien Ermessen des auf diesem Gebiet tätigen Fachmanns.

Im Rahmen der vorliegenden Erfindung kann die Bestimmung der Osmolalität der erfindungsgemäßen Zusammensetzung mit dem Fachmann an sich bekannten Methoden erfolgen. Insbesondere kann die Bestimmung der Osmolalität der erfindungsgemäßen Zusammensetzung gemäß der Methode nach Ph. Eur. [Pharmacopoea Europaea] 7. Ausgabe, Grundwerk 2011, Abschnitt 2.2.35, Kap. 5.3.9, erfolgen.

Ebenfalls kann die Dichte der cineolhaltigen Zusammensetzung nach der vorliegenden Erfindung in weiten Bereichen variieren. Insbesondere kann es vorgesehen sein, dass die erfindungsgemäße cineolhaltige Zusammensetzung bei einer Temperatur von 20 °C und bei einem Druck von 1.013,25 mbar (Atmosphärendruck) eine relative Dichte, bezogen auf reines Wasser, im Bereich von 1,001 bis 1,2, insbesondere im Bereich von 1,005 bis 1,15, vorzugsweise im Bereich von 1,005 bis 1,105, aufweist. Dennoch kann es einzelfallbedingt oder anwendungsbezogen erforderlich sein, von den vorgenannten Werten abzuweichen, ohne dass der Rahmen der vorliegenden Erfindung verlassen ist.

Im Rahmen der vorliegenden Erfindung kann die Bestimmung der relativen Dichte der erfindungsgemäßen Zusammensetzung mit dem Fachmann an sich bekannten Methoden erfolgen. Insbesondere kann die Bestimmung der relativen Dichte der erfindungsgemäßen Zusammensetzung gemäß der Methode nach Ph. Eur. [Pharmacopoea Europaea] 7. Ausgabe, Grundwerk 2011, Abschnitt 2.2.5, erfolgen.

Anwendungsabhängig kann auch die Viskosität der cineolhaltigen Zusammensetzung nach der vorliegenden Erfindung in weiten Bereichen eingestellt bzw. ausgewählt sein.

Gemäß einer besonderen Ausführungsform, wonach die erfindungsgemäße Zusammensetzung in Abwesenheit eines Verdickungsmittels vorliegt (d. h. wenn die erfindungsgemäße Zusammensetzung ohne ein Verdickungsmittel formuliert ist bzw. kein Verdickungsmittel enthält), kann die Zusammensetzung nach der vorliegenden Erfindung bei einer Temperatur von 20 °C eine dynamische Viskosität im Bereich von 1,001 bis 2 mPas, insbesondere 1,01 bis 1,9 mPas, vorzugsweise 1,05 bis 1,8 mPas, besonders bevorzugt 1,1 bis 1,5 mPas, aufweisen (wobei die Bestimmung der dynamischen Viskosität insbesondere gemäß der Methode nach Ph. Eur. [Pharmacopoea Europaea] 7. Ausgabe, Grundwerk 2011, Abschnitt 2.2.9 Kapillarviskosimeter [bevorzugt Ubbelohde-Viskosimeter], erfolgen kann). Eine solche Ausführungsform ist insbesondere dann bevorzugt, wenn eine gute Applizierbarkeit der Zusammensetzung mittels Sprühauftrag im Vordergrund steht.

Gemäß einer weiteren, hierzu alternativen besonderen Ausführungsform, wonach die erfindungsgemäße Zusammensetzung in Anwesenheit eines Verdickungsmittels vorliegt (d. h. wenn die erfindungsgemäße Zusammensetzung mit einem Verdickungsmittel formuliert ist bzw. ein Verdickungsmittel enthält), kann die Zusammensetzung nach der vorliegenden Erfindung bei einer Temperatur von 20 °C eine dynamische Viskosität von mindestens 1,5 mPas, insbesondere im Bereich von 1,5 bis 10⁵ mPas, vorzugsweise 1,6 bis 10⁴ mPas, besonders bevorzugt 1,7 bis 10³ mPas, noch mehr bevorzugt 2 bis 100 mPas, aufweisen (wobei die Bestimmung der dynamischen Viskosität insbesondere gemäß der Methode nach Ph. Eur. [Pharmacopoea Europaea] 7. Ausgabe, Grundwerk 2011, Abschnitt 2.2.9 Kapillarviskosimeter [bevorzugt Ubbelohde-Viskosimeter], erfolgen kann). Eine solche Ausführungsform ist insbesondere dann bevorzugt, wenn eine verlängerte Wirkungsdauer und/oder eine verbesserte Bioadhäsion der Zusammensetzung im Vordergrund steht.

Ebenfalls kann der Brechungsindex der cineolhaltigen Zusammensetzung nach der vorliegenden Erfindung in weiten Bereichen variieren. Insbesondere kann es vorgesehen sein, dass die erfindungsgemäße cineolhaltige Zusammensetzung bei einer Temperatur von 20 °C und bei einem Druck von 1.013,25 mbar (Atmosphärendruck) einen Brechungsindex im Bereich von 1,10 bis 1,50, insbesondere im Bereich von 1,20 bis 1,40, vorzugsweise im Bereich von 1,25 bis 1,39, aufweist. Dennoch kann es einzelfallbedingt oder anwendungsbezogen erforderlich sein, von den vorgenannten Werten abzuweichen, ohne dass der Rahmen der vorliegenden Erfindung verlassen ist.

Im Rahmen der vorliegenden Erfindung kann die Brechungsindexbestimmung der erfindungsgemäßen Zusammensetzung mit dem Fachmann an sich bekannten Methoden erfolgen. Insbesondere kann die Brechungsindexbestimmung der erfindungsgemäßen Zusammensetzung gemäß der Methode nach Ph. Eur. [Pharmacopoea Europaea] 7. Ausgabe, Grundwerk 2011, Abschnitt 2.2.6, erfolgen.

Wie zuvor bereits ausgeführt, verfügt die cineolhaltige Zusammensetzung nach der vorliegenden Erfindung über eine gute Stabilität, insbesondere Lager- und Langzeitstabilität. Gemäß einer besonderen Ausführungsform ist die Zusammensetzung bei Temperaturen im Bereich von 20 °C bis 50 °C, bei einem Druck von 1.013,25 mbar (Atmosphärendruck) und bei einer relativen Luftfeuchtigkeit im Bereich von 50 % bis 90 % mindestens 6 Monate, insbesondere mindestens 12 Monate, vorzugsweise mindestens 24 Monate, bevorzugt mindestens 36 Monate, stabil, insbesondere lagerstabil, ausgebildet.

Des Weiteren kann es erfindungsgemäß vorgesehen sein, dass die erfindungsgemäße cineolhaltige Zusammensetzung mindestens einen weiteren Inhaltsstoff, insbesondere Hilfsstoff und/oder Additiv, aufweist. Der weitere Inhaltsstoff, insbesondere Hilfsstoff und/oder Additiv, kann insbesondere ausgewählt sein aus der Gruppe von Verarbeitungshilfsstoffen, Stabilisatoren, Emulgatoren, Antioxidantien, Feuchthaltemitteln, Verdickungsmitteln, Antiseptika, Farbstoffen, Aromastoffen, Riechstoffen, Duftstoffen, Streckmitteln, Bindemitteln, Netzmitteln, Vitaminen, Spurenelementen, Mineralstoffen, Mikronährstoffen und/oder ätherischen Ölen sowie deren Kombinationen (d. h. Kombinationen von zwei oder mehreren der vorgenannten Inhaltsstoffen).

Wie eingangs dargelegt, verfügt die cineolhaltige Zusammensetzung nach der vorliegenden Erfindung über ein breites Anwendungsprofil. Insbesondere betrifft die vorliegende Erfindung eine wie zuvor geschilderte cineolhaltige Zusammensetzung zur Verwendung bei der prophylaktischen und/oder kurativen topischen Behandlung von Rhinitiden, insbesondere von *Rhinitis acuta.*

Des Weiteren betrifft die vorliegende Erfindung - gemäß einem **zweiten** Aspekt der vorliegenden Erfindung - eine Applikationsvorrichtung, welche eine wie zuvor beschriebene cineolhaltige Zusammensetzung nach der vorliegenden Erfindung enthält, wobei die erfindungsgemäße Applikationsvorrichtung insbesondere für die topische, insbesondere nasale, vorzugsweise intranasale Applikation geeignet ist und bevorzugt in Form eines Behältnisses mit Tropf- oder Sprüheinrichtung vorliegt.

Erfindungsgemäß bevorzugt ist es, wenn die erfindungsgemäße Applikationsvorrichtung eine Sprüheinrichtung zur vorzugsweise gleichförmigen Ausbringung der Zusammensetzung nach der vorliegenden Erfindung in einer Menge pro Sprühstoß im Bereich von 25 µl bis 300 µl, insbesondere im Bereich von 50 µl bis 200 µl, vorzugsweise im Bereich von 75 µl bis 125 µl, aufweist.

Dabei ist es insbesondere vorgesehen, dass die erfindungsgemäße Applikationsvorrichtung ein Behältnis bzw. einen Vorratsbehälter mit einem Volumen im Bereich von 5 ml bis 100 ml, insbesondere 10 ml bis 50 ml, aufweist. Dieses Behältnis bzw. dieser Vorratsbehälter dient dann zur Aufnahme der erfindungsgemäßen Zusammensetzung.

Für weitergehende Einzelheiten zu der erfindungsgemäßen Applikationsvorrichtung kann auf die vorangehenden Ausführungen zu der erfindungsgemäßen Zusammensetzung verwiesen werden, welche im Hinblick auf die erfindungsgemäße Applikationsvorrichtung entsprechend gelten.

Darüber hinaus eignet sich die erfindungsgemäße Zusammensetzung zur Verwendung bei der prophylaktischen und/oder kurativen topischen Behandlung von Rhinitiden aller Art, insbesondere von *Rhinitis acuta,* bzw. zur Verwendung bei der Herstellung eines Arzneimittels zur prophylaktischen und/oder kurativen topischen Behandlung von Rhinitiden aller Art, insbesondere von *Rhinitis acuta.*

Der Begriff des Arzneimittels (synonym auch Pharmazeutikum), wie er im Rahmen der vorliegenden Erfindung verwendet wird, ist sehr umfänglich zu verstehen und umfasst nicht nur Arzneimittel bzw. Pharmazeutika als solche (d. h. in arzneimittelrechtlicher Hinsicht), sondern vor allem auch sogenannte Medizinprodukte und darüber hinaus aber auch Homöopathika und Nahrungsergänzungsmittel sowie Kosmetika und Gebrauchsgegenstände. Mit anderen Worten kann also die erfindungsgemäße cineolhaltige Zusammensetzung in Form eines Arzneimittels (Pharmazeutikums), Medizinprodukts, Homöopathikums, Nahrungsergänzungsmittels, Kosmetikums oder Gebrauchsgegenstands vorliegen.

Weiterer Gegenstand der vorliegenden Erfindung - gemäß einem **dritten** Aspekt der vorliegenden Erfindung - ist die Verwendung der erfindungsgemäßen Zusammensetzung zur Behandlung von Rhinitiden aller Art, insbesondere *Rhinitis acuta, Rhinitis allergica, Rhinitis atrophicans, Rhinitis hyperplastica* oder *hypertrophicans, Rhinitis mutilans, Rhinitis nervosa* oder *vasomotorica,* umweltbedingter Rhinitis oder *Rhinitis pseudomembranacea,* vorzugsweise *Rhinitis acuta.*

Wie zuvor beschrieben, kann im Rahmen der Verwendung die erfindungsgemäße Zusammensetzung, wie zuvor beschrieben, topisch, insbesondere nasal, vorzugsweise intranasal, appliziert werden. Dabei kann insbesondere die erfindungsgemäße Applikationseinrichtung, wie sie zuvor beschrieben worden ist, zur Anwendung kommen.

Die folgenden Ausführungsbeispiele dienen lediglich der Veranschaulichung der vorliegenden Erfindung, ohne die vorliegende Erfindung jedoch hierauf zu beschränken.

### AUSFÜHRUNGESBEISPIELE

### 1. Herstellungsbeispiele

### Allgemeine Herstellvorschrift

Zur Herstellung von jeweils 1.000 g einer klaren wässrigen Lösung der erfindungsgemäßen Zusammensetzung wird in dem Fachmann an sich bekannter Weise vorgegangen: Zunächst wird bei Raumtemperatur und Umgebungsdruck in einem entsprechenden Glasgefäß mit Rühreinrichtung eine definierte Menge an gereinigtem Wasser vorgelegt und nachfolgend mit einer Lösung des gewählten Puffersystems auf einen vorgegeben pH-Wert im Bereich zwischen 5,3 und 5,9 (z. B. pH-Wert von ca. 5,5) unter anschließender Kontrolle des erreichten pH-Werts eingestellt. Anschließend werden hierzu die nachfolgenden in den Rezepturbeispielen spezifizierten Mengen der weiteren Wirk- und Inhaltsstoffe (1,8-Cineol, Dexpanthenol sowie gegebenenfalls z.B. Emulgator, Konservierungs-/Desinfektionsmittel (Antiseptikum), Verdickungsmittel etc.) hinzugegeben, und das Ganze wird dann durch gründliches Rühren klar gelöst. Nachfolgend wird das Ganze mit weiterem Wasser zum Endgewicht von 1.000 g aufgefüllt und homogen gerührt. Gegebenenfalls wird die Lösung über neutrale Cellulosefilter filtriert. Abschließend wird der pH-Wert noch einmal kontrolliert. Auch die übrigen entsprechenden Spezifikationen der Lösung werden auf die eingestellten bzw. vorgewählten Wertebereiche hin überprüft (z. B. Osmolalität; relative Dichte; mikrobiologische Reinheit und Sterilität; Ausschluss von Verunreinigungen, insbesondere Abbauprodukten der Inhalts- und Wirkstoffe; Viskosität; Aussehen; Brechungsindex). Ein Teil der erhaltenen Lösung wird schließlich in Enghalsflaschen aus Braunglas zu 10 ml oder 20 ml abgefüllt, welche wahlweise mit einer Tropfpipette oder einer Sprühdosierpumpe ausgerüstet werden können; zur bestimmungsgemäßen Anwendung können mehrmals täglich ein bis drei Tropfen bzw. ein bis zwei Sprühstöße in jedes Nasenloch gegeben und aufgezogen werden. Ein anderer Teil der erhaltenen Lösung wird für Stabilitätsuntersuchungen verwendet.

Nach dieser allgemeinen Herstellvorschrift werden die nachfolgend spezifizierten Rezepturen hergestellt. Alle Rezepturen sind über 36 Monate lagerstabil.

**Rezeptur 1 (Angabe pro 1.000 g Zusammensetzung)**

| Inhaltsstoff | Menge / Gew.-% | Qualität |
|---|---|---|
| Cineol | 2,000 | Ph. Eur. |
| Dexpanthenol | 5,000 | Ph. Eur. |
| Puffersystem: | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) (Benzalkoniumchlorid) | 0,040 | Ph. Eur. |
| Emulgator (PEG-40-ethoxyliertes und hydriertes Ricinusöl) | 0,200 | Ph. Eur. |
| Gereinigtes Wasser | 91,880 | Ph. Eur. |

| | | |
|---|---|---|
| Viskosität (20 °C): 1,10-1,20 mPas | | |

**Rezeptur 2 (Angabe pro 1.000 g Zusammensetzung)**

| Inhaltsstoff | Menge / Gew.-% | Qualität |
|---|---|---|
| Cineol | 2,000 | Ph. Eur. |
| Dexpanthenol | 5,000 | Ph. Eur. |
| Puffersystem : | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) (Benzalkoniumchlorid) | 0,040 | Ph. Eur. |
| Emulgator (PEG-40-ethoxyliertes und hydriertes Ricinusöl) | 4,000 | Ph. Eur. |
| Gereinigtes Wasser | 88,080 | Ph. Eur. |

| | | |
|---|---|---|
| Viskosität (20 °C): 1,20-1,25 mPas | | |

**Rezeptur 3 (Angabe pro 1.000 g Zusammensetzung)**

| Inhaltsstoff | Menge / Gew.-% | Qualität |
|---|---|---|
| Cineol | 1,000 | Ph. Eur. |
| Dexpanthenol | 5,000 | Ph. Eur. |
| Puffersystem: | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) (Benzalkoniumchlorid) | 0,040 | Ph. Eur. |
| Emulgator (PEG-40-ethoxyliertes und hydriertes Ricinusöl) | 2,000 | Ph. Eur. |
| Gereinigtes Wasser | 91,080 | Ph. Eur. |

| | | |
|---|---|---|
| Viskosität (20 °C): 1,18 mPas | | |

**Rezeptur 4 (Angabe pro 1.000 g Zusammensetzung)**

| Inhaltsstoff | Menge / Gew.-% | Qualität |
|---|---|---|
| Cineol | 0,100 | Ph. Eur. |
| Dexpanthenol | 5,000 | Ph. Eur. |
| Puffersystem : | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) (Benzalkoniumchlorid) | 0,040 | Ph. Eur. |
| Emulgator (PEG-40-ethoxyliertes und hydriertes Ricinusöl) | 0,200 | Ph. Eur. |
| Gereinigtes Wasser | 93,780 | Ph. Eur. |

| | | |
|---|---|---|
| Viskosität (20 °C): 1,15-1,20 mPas | | |

**Rezeptur 5 (Angabe pro 1.000 g Zusammensetzung)**

| Inhaltsstoff | Menge / Gew.-% | Qualität |
|---|---|---|
| Cineol | 0,600 | Ph. Eur. |
| Dexpanthenol | 5,000 | Ph. Eur. |
| Puffersystem : | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) (Benzalkoniumchlorid) | 0,040 | Ph. Eur. |
| Emulgator (PEG-40-ethoxyliertes und hydriertes Ricinusöl) | 1,200 | Ph. Eur. |
| Gereinigtes Wasser | 92,880 | Ph. Eur. |

| | | |
|---|---|---|
| Viskosität (20 °C): 1,10-1,22 mPas | | |

**Rezeptur 6 (Angabe pro 1.000 g Zusammensetzung)**

| Inhaltsstoff | Menge / Gew.-% | Qualität |
|---|---|---|
| Cineol | 0,600 | Ph. Eur. |
| Dexpanthenol | 5,000 | Ph. Eur. |
| Puffersystem: | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) (Benzalkoniumchlorid) | 0,040 | Ph. Eur. |
| Emulgator (PEG-40-ethoxyliertes und hydriertes Ricinusöl) | 1,200 | Ph. Eur. |
| Aromastoff mit Kältevermittlung (wasserlösliche kühlende Aromen oder entsprechende ätherische Öle) | 0,040 | Ph. Eur. |
| Gereinigtes Wasser | 92,240 | Ph. Eur. |

| | | |
|---|---|---|
| Viskosität (20 °C): 1,10-1,22 mPas | | |

**Rezeptur 7 (Angabe pro 1.000 g Zusammensetzung)**

| Inhaltsstoff | Menge / Gew.-% | Qualität |
|---|---|---|
| Cineol | 0,600 | Ph. Eur. |
| Dexpanthenol | 5,000 | Ph. Eur. |
| Puffersystem: | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) (Benzalkoniumchlorid) | 0,040 | Ph. Eur. |
| Emulgator (PEG-40-ethoxyliertes und hydriertes Ricinusöl) | 1,200 | Ph. Eur. |
| Aromastoff mit Kältevermittlung (wasserlösliche kühlende Aromen oder entsprechende ätherische Öle) | 0,080 | Ph. Eur. |
| Gereinigtes Wasser | 92,200 | Ph. Eur. |

| | | |
|---|---|---|
| Viskosität (20 °C): 1,10-1,22 mPas | | |

**Rezeptur 8 (Angabe pro 1.000 g Zusammensetzung)**

| Inhaltsstoff | Menge / Gew.-% | Qualität |
|---|---|---|
| Cineol | 0,600 | Ph. Eur. |
| Dexpanthenol | 5,000 | Ph. Eur. |
| Puffersystem: | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) (Benzalkoniumchlorid) | 0,040 | Ph. Eur. |
| Emulgator (Undecylenamidopropylbetain) | 0,100 | Ph. Eur. |
| Gereinigtes Wasser | 93,380 | Ph. Eur. |

| | | |
|---|---|---|
| Viskosität (20 °C): 1,10-1,22 mPas | | |

**Rezeptur 9 (Angabe pro 1.000 g Zusammensetzung)**

| Inhaltsstoff | Menge / Gew.-% | Qualität |
|---|---|---|
| Cineol | 0,600 | Ph. Eur. |
| Dexpanthenol | 5,000 | Ph. Eur. |
| Puffersystem: | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) (Polyhexanid) | 0,100 | Ph. Eur. |
| Emulgator (Undecylenamidopropylbetain) | 0,100 | Ph. Eur. |
| Gereinigtes Wasser | 93,320 | Ph. Eur. |

| | | |
|---|---|---|
| Viskosität (20 °C): 1,10-1,22 mPas | | |

**Rezeptur 10 (Angabe pro 1.000 g Zusammensetzung)**

| Inhaltsstoff | Menge / Gew.-% | Qualität |
|---|---|---|
| Cineol | 0,600 | Ph. Eur. |
| Dexpanthenol | 5,000 | Ph. Eur. |
| Puffersystem: | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) (Polyhexanid) | 0,100 | Ph. Eur. |
| Emulgator (Undecylenamidopropylbetain) | 0,100 | Ph. Eur. |
| Verdickungsmittel (Poloxamer) | 5,000 | Ph. Eur. |
| Gereinigtes Wasser | 88,320 | Ph. Eur. |

| | | |
|---|---|---|
| Viskosität (20 °C): 10³ mPas | | |

**Rezeptur 11 (Angabe pro 1.000 g Zusammensetzung)**

| Inhaltsstoff | Menge / Gew.-% | Qualität |
|---|---|---|
| Cineol | 0,600 | Ph. Eur. |
| Dexpanthenol | 5,000 | Ph. Eur. |
| Puffersystem: | | Ph. Eur. |
| Kaliumdihydrogenphosphat / | 0,853 | |
| Natriummonohydrogenphosphat (Dodekahydrat) | 0,027 | |
| Emulgator (Undecylenamidopropylbetain) | 0,100 | Ph. Eur. |
| Vitamine A und C (Micelle) | 1,000 | Ph. Eur. |
| Gereinigtes Wasser | 92,420 | Ph. Eur. |

| | | |
|---|---|---|
| Viskosität (20 °C): 1,15-1,25 mPas | | |

**Rezeptur 12 (Angabe pro 1.000 g Zusammensetzung)**

| Inhaltsstoff | Menge / Gew.-% | Qualität |
|---|---|---|
| Cineol (Liposomen) | 0,600 | Ph. Eur. |
| Dexpanthenol | 5,000 | Ph. Eur. |
| Puffersystem : | | Ph. Eur. |
| Kaliumdihydrogenphosphat / | 0,853 | |
| Natriummonohydrogenphosphat (Dodekahydrat) | 0,027 | |
| Emulgator (Undecylenamidopropylbetain) | 0,100 | Ph. Eur. |
| Gereinigtes Wasser | 93,420 | Ph. Eur. |

| | | |
|---|---|---|
| Viskosität (20 °C): 1,20-1,30 mPas | | |

**Rezeptur 13 (Angabe pro 1.000 g Zusammensetzung)**

| Inhaltsstoff | Menge / Gew.-% | Qualität |
|---|---|---|
| Cineol | 0,600 | Ph. Eur. |
| Dexpanthenol | 5,000 | Ph. Eur. |
| Puffersystem : | | Ph. Eur. |
| Kaliumdihydrogenphosphat / | 0,853 | |
| Natriummonohydrogenphosphat (Dodekahydrat) | 0,027 | |
| Emulgator (Undecylenamidopropylbetain) | 0,100 | Ph. Eur. |
| Verdickungsmittel (Natriumhyaluronat) | 0,020 | Ph. Eur. |
| Konservierungs-/Desinfektionsmittel (Antiseptikum) (Kaliumsorbat) | 0,020 | Ph. Eur. |
| Gereinigtes Wasser | 93,380 | Ph. Eur. |

| | | |
|---|---|---|
| Viskosität (20 °C): 5 • 10² mPas | | |

**Rezeptur 14 (Angabe pro 1.000 g Zusammensetzung)**

| Inhaltsstoff | Menge / Gew.-% | Qualität |
|---|---|---|
| Cineol | 0,600 | Ph. Eur. |
| Dexpanthenol | 5,000 | Ph. Eur. |
| Puffersystem: | | Ph. Eur. |
| Kaliumdihydrogenphosphat / | 0,853 | |
| Natriummonohydrogenphosphat (Dodekahydrat) | 0,027 | |
| Emulgator (Undecylenamidopropylbetain) | 0,100 | Ph. Eur. |
| Verdickungsmittel (Carrageenan) | 0,400 | Ph. Eur. |
| Gereinigtes Wasser | 93,020 | Ph. Eur. |

| | | |
|---|---|---|
| Viskosität (20 °C): 10³ mPas | | |

**Rezeptur 15 (Angabe pro 1.000 g Zusammensetzung)**

| Inhaltsstoff | Menge / Gew.-% | Qualität |
|---|---|---|
| Cineol | 0,600 | Ph. Eur. |
| Dexpanthenol | 5,000 | Ph. Eur. |
| Xylometazolinhydrochlorid | 0,050 | Ph. Eur. |
| Puffersystem : | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) (Benzalkoniumchlorid) | 0,050 | Ph. Eur. |
| Emulgator (Poloxamer) | 1,250 | Ph. Eur. |
| Verdickungsmittel (Natriumalginat) | 1,500 | Ph. Eur. |
| Gereinigtes Wasser | 90,670 | Ph. Eur. |

| | | |
|---|---|---|
| Viskosität (20 °C): 1,5 • 10³ mPas | | |

**Rezeptur 16 (Angabe pro 1.000 g Zusammensetzung)**

| Inhaltsstoff | Menge / Gew.-% | Qualität |
|---|---|---|
| Cineol | 1,000 | Ph. Eur. |
| Dexpanthenol | 5,000 | Ph. Eur. |
| Oxymetazolinhydrochlorid | 0,100 | Ph. Eur. |
| Puffersystem : | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) (Benzalkoniumchlorid) | 0,050 | Ph. Eur. |
| Emulgator (Poloxamer) | 1,250 | Ph. Eur. |
| Gereinigtes Wasser | 91,720 | Ph. Eur. |

| | | |
|---|---|---|
| Viskosität (20 °C): 1,25-1,35 mPas | | |

**Rezeptur 17 (Angabe pro 1.000 g Zusammensetzung)**

| Inhaltsstoff | Menge / Gew.-% | Qualität |
|---|---|---|
| Cineol | 1,000 | Ph. Eur. |
| Natriumpantothenat | 5,000 | Ph. Eur. |
| Oxymetazolinhydrochlorid | 0,100 | Ph. Eur. |
| Puffersystem: | | Ph. Eur. |
| Kaliumdihydrogenphosphat / | 0,853 | |
| Natriummonohydrogenphosphat (Dodekahydrat) | 0,027 | |
| Konservierungs-/Desintektionsmittel (Antiseptikum) (Benzalkoniumchlorid) | 0,050 | Ph. Eur. |
| Emulgator (Poloxamer) | 1,250 | Ph. Eur. |
| Gereinigtes Wasser | 91,720 | Ph. Eur. |

| | | |
|---|---|---|
| Viskosität (20 °C): 1,25-1,35 mPas | | |

**Rezeptur 18 (Angabe pro 1.000 g Zusammensetzung)**

| Inhaltsstoff | Menge / Gew.-% | Qualität |
|---|---|---|
| Cineol | 1,000 | Ph. Eur. |
| Dexpanthenol | 5,000 | Ph. Eur. |
| Oxymetazolinhydrochlorid | 0,050 | Ph. Eur. |
| Puffersystem: | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) (Benzalkoniumchlorid) | 0,050 | Ph. Eur. |
| Emulgator (Poloxamer) | 1,250 | Ph. Eur. |
| Gereinigtes Wasser | 91,770 | Ph. Eur. |

| | | |
|---|---|---|
| Viskosität (20 °C): 1,25-1,30 mPas | | |

**Rezeptur 19 (Angabe pro 1.000 g Zusammensetzung)**

| Inhaltsstoff | Menge / Gew.-% | Qualität |
|---|---|---|
| Cineol | 1,000 | Ph. Eur. |
| Natriumpantothenat | 5,000 | Ph. Eur. |
| Oxymetazolinhydrochlorid | 0,050 | Ph. Eur. |
| Puffersystem : | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) (Benzalkoniumchlorid) | 0,050 | Ph. Eur. |
| Emulgator (Poloxamer) | 1,250 | Ph. Eur. |
| Gereinigtes Wasser | 91,770 | Ph. Eur. |

| | | |
|---|---|---|
| Viskosität (20 °C): 1,30-1,35 mPas | | |

**Rezeptur 20 (Angabe pro 1.000 g Zusammensetzung)**

| Inhaltsstoff | Menge / Gew.-% | Qualität |
|---|---|---|
| Cineol | 0,600 | Ph. Eur. |
| Dexpanthenol | 5,000 | Ph. Eur. |
| Xylometazolinhydrochlorid | 0,100 | Ph. Eur. |
| Puffersystem : | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) (Benzalkoniumchlorid) | 0,050 | Ph. Eur. |
| Emulgator (Poloxamer) | 1,250 | Ph. Eur. |
| Verdickungsmittel (Natriumalginat) | 1,500 | Ph. Eur. |
| Gereinigtes Wasser | 90,620 | Ph. Eur. |

| | | |
|---|---|---|
| Viskosität (20 °C): 1,5 • 10³ mPas | | |

**Rezeptur 21 (Angabe pro 1.000 g Zusammensetzung)**

| Inhaltsstoff | Menge / Gew.-% | Qualität |
|---|---|---|
| Cineol | 0,600 | Ph. Eur. |
| Dexpanthenol | 5,000 | Ph. Eur. |
| Puffersystem : | | Ph. Eur. |
| Kaliumdihydrogenphosphat / | 0,008 | |
| Natriummonohydrogenphosphat (Dodekahydrat) | 0,100 | |
| Natriumchlorid | 3,000 | Ph. Eur. |
| Emulgator (Undecylenamidopropylbetain) | 0,100 | Ph. Eur. |
| Gereinigtes Wasser | 91,192 | Ph. Eur. |

| | | |
|---|---|---|
| Viskosität (20 °C): 1,30-1,35 mPas | | |

**Rezeptur 22 (Angabe pro 1.000 g Zusammensetzung)**

| Inhaltsstoff | Menge / Gew.-% | Qualität |
|---|---|---|
| Cineol | 0,600 | Ph. Eur. |
| Dexpanthenol | 5,000 | Ph. Eur. |
| Puffersystem: | | Ph. Eur. |
| Kaliumdihydrogenphosphat / | 0,008 | |
| Natriummonohydrogenphosphat (Dodekahydrat) | 0,100 | |
| Natriumchlorid | 0,900 | Ph. Eur. |
| Emulgator (Undecylenamidopropylbetain) | 0,100 | Ph. Eur. |
| Gereinigtes Wasser | 93,292 | Ph. Eur. |

| | | |
|---|---|---|
| Viskosität (20 °C): 1,25-1,30 mPas | | |

**Rezeptur 23 (Angabe pro 1.000 g Zusammensetzung)**

| Inhaltsstoff | Menge / Gew.-% | Qualität |
|---|---|---|
| Cineol | 0,600 | Ph. Eur. |
| Dexpanthenol | 5,000 | Ph. Eur. |
| Puffersystem : | | Ph. Eur. |
| Kaliumdihydrogenphosphat / | 0,008 | |
| Natriummonohydrogenphosphat (Dodekahydrat) | 0,100 | |
| Ectoin | 2,000 | Ph. Eur. |
| Emulgator (Undecylenamidopropylbetain) | 0,100 | Ph. Eur. |
| Gereinigtes Wasser | 92,192 | Ph. Eur. |

| | | |
|---|---|---|
| Viskosität (20 °C): 1,35-1,40 mPas | | |

**Rezeptur 24 (Angabe pro 1.000 g Zusammensetzung)**

| Inhaltsstoff | Menge / Gew.-% | Qualität |
|---|---|---|
| Cineol | 0,600 | Ph. Eur. |
| Dexpanthenol | 5,000 | Ph. Eur. |
| Puffersystem: | | Ph. Eur. |
| Kaliumdihydrogenphosphat / | 0,008 | |
| Natriummonohydrogenphosphat (Dodekahydrat) | 0,100 | |
| Ectoin | 2,000 | Ph. Eur. |
| Emulgator (Undecylenamidopropylbetain) | 0,100 | Ph. Eur. |
| Xylometazolinhydrochlorid | 0,100 | Ph. Eur. |
| Gereinigtes Wasser | 92,092 | Ph. Eur. |

| | | |
|---|---|---|
| Viskosität (20 °C): 1,38-1,40 mPas | | |

### .2 Stabilitätsuntersuchungen und weitere Untersuchungen

### a) Stabilitätsverhalten in Abhängigkeit von pH-Wert

Entsprechend der vorstehenden allgemeinen Herstellvorschrift werden wässrige Zusammensetzungen nach der vorliegenden Erfindung jeweils mit 0,600 Gew.-% 1,8-Cineol und 5,000 Gew.-% Dexpanthenol (jeweils bezogen auf die Zusammensetzung) hergestellt.

Mit unterschiedlichen Mengen an Puffer und erforderlichenfalls unterschiedlichen Puffersystemen wird jeweils der pH-Wert der Zusammensetzungen variiert und das Stabilitätsverhalten untersucht (Lagerung jeweils bei 25 °C, bei einem Druck von 1.013,25 mbar [Atmosphärendruck] und bei einer relativen Luftfeuchtigkeit von 75 %). Bei Zusammensetzungen mit einem pH-Wert außerhalb des Bereichs von 5 bis 6,5 handelt es sich um nichterfindungsgemäße Vergleichszusammensetzungen.

Die nachfolgenden Angaben beziehen sich auf die maximale Lagerfähigkeit bei den unterschiedlichen angegeben pH-Werten (Anforderung an die Stabilität: Abbau Cineol ≤ 1 %, bezogen auf Cineol; Abbau Dexpanthenol ≤ 1 %, bezogen auf Dexpanthenol):

| pH-Wert der Zusammensetzung | Lagerfähigkeit |
|---|---|
| 3,0 | 27 Monate |
| 4,0 | 26 Monate |
| 4,5 | 28,5 Monate |
| 5,0 | 30,5 Monate |
| 5,3 | > 36 Monate |
| 5,5 | > 36 Monate |
| 5,9 | > 36 Monate |
| 6,5 | 30,5 Monate |
| 7,5 | 29,5 Monate |
| 8,0 | 26,5 Monate |
| 9,0 | 23 Monate |

Wie die Untersuchungen zeigen, wird die beste Lagerfähigkeit in einem pH-Wertebereich von 5,0 bis 6,5, insbesondere 5,3 bis 5,9, erreicht. Bei diesen Zusammensetzungen wird ein wie zuvor spezifiziertes pH-Wertpuffersystem auf Phosphatbasis (d. h. Dihydrogenphosphat/Monohydrogenphosphat-Puffersystem bzw. H₂PO₄⁻/HPO₄²⁻-Puffer) eingesetzt.

### b) Stabilitätsverhalten in Abhängigkeit von weiteren Inhaltsstoffen

Entsprechend der vorstehenden allgemeinen Herstellvorschrift werden wässrige Zusammensetzungen ("Zusammensetzungen A1 bis A6") nach der vorliegenden Erfindung jeweils mit 0,800 Gew.-% 1,8-Cineol und 4,750 Gew.-% Dexpanthenol (jeweils bezogen auf die Zusammensetzung) hergestellt. Mit einem wie zuvor spezifizierten pH-Wertpuffersystem auf Phosphatbasis wird jeweils ein pH-Wert im Bereich von 5,3 bis 5,9 eingestellt (0,853 Gew.-% Kaliumdihydrogenphosphat und 0,027 Gew.-% Natriummonohydrogenphosphat-Dodekahydrat, jeweils bezogen auf die Zusammensetzung).

Eine erste erfindungsgemäße Zusammensetzung ("Zusammensetzung A1") enthält keine weitergehenden Inhaltsstoffe als die vorgenannten Inhaltsstoffe.

Den übrigen erfindungsgemäßen Zusammensetzungen ("Zusammensetzungen A2 bis A6") werden zusätzlich weitere Inhaltsstoffe (d. h. Emulgator [polyethoxyliertes hydriertes Ricinusöl] und/oder Konservierungs-/Desinfektionsmittel [Benzalkoniumchlorid] und/oder Verdickungsmittel [Natriumhyaluronat]) zugesetzt, und zwar wie nachfolgend angegeben (Zusammensetzung A2: 1,250 Gew.-% Emulgator; Zusammensetzung A3: 0,050 Gew.-% Konservierungs-/Desinfektionsmittel; Zusammensetzung A4: 1,250 Gew.-% Emulgator und 0,050 Gew.-% Konservierungs-/Desinfektionsmittel; Zusammensetzung A5: 1,500 Gew.-% Verdickungsmittel; Zusammensetzung A6: 1,250 Gew.-% Emulgator, 0,050 Gew.-% Konservierungs-/ Desinfektionsmittel und 1,500 Gew.-% Verdickungsmittel).

Das Stabilitätsverhalten dieser Zusammensetzungen wird unter definierten Bedingungen untersucht (Lagerung jeweils bei 25 °C, bei einem Druck von 1.013,25 mbar [Atmosphärendruck] und bei einer relativen Luftfeuchtigkeit von 75 %). Die nachfolgenden Angaben beziehen sich auf die maximale Lagerfähigkeit (Anforderung an die Stabilität: Abbau Cineol ≤ 1 %, bezogen auf Cineol; Abbau Dexpanthenol ≤ 1 %, bezogen auf Dexpanthenol):

| Zusammensetzung | Lagerfähigkeit |
|---|---|
| A1 | 37,5 Monate |
| A2 | 40,5 Monate |
| A3 | 39,5 Monate |
| A4 | 44,5 Monate |
| A5 | 38,5 Monate |
| A6 | 45,5 Monate |

Wie die Untersuchungen zeigen, werden besonders gute Lagerfähigkeiten erhalten, wenn den erfindungsgemäßen Zusammensetzungen neben den eigentlichen Wirkstoffen (Cineol und Dexpanthenol) weitere Inhaltsstoffe auf Basis von Emulgatoren, Konservierungs-/Desinfektionsmitteln und/oder Verdickungsmitteln zugesetzt werden.

### c) Untersuchungen zum antibakteriellen und antiviralen Wirkverhalten

Die in dem vorstehenden Ausführungsbeispiel 2 b) beschriebenen erfindungsgemäßen Zusammensetzungen A1 bis A6 werden auf ihr antibakterielles und antivirales Wirkverhalten getestet. Eine weitere, aber nichterfindungsgemäße Zusammensetzung A7 wird getestet, wobei die nichterfindungsgemäße Zusammensetzung A7 der erfindungsgemäßen Zusammensetzung A1 entspricht, wobei jedoch das Cineol durch einen entsprechenden Wasseranteil ersetzt ist.

Die antibakterielle Aktivität der Zusammensetzungen wird in einem Plattendiffusionstest sowie in einer Verdünnungstestreihe an 18 Bakterienstämmen (hierunter *Staphylococcus aureus, Haemophilus influenzae, Moraxella catarrhalis, Streptococcus pneumoniae, Bacillus sp., Citrobacter sp., Escherichia coli, Klebsiella sp., Salmonella sp.* und *Vibrio cholera*) getestet (Ermittlung der MIC-Konzentrationen [*Mininimal Inhibitory Concentrations* bzw. minimale Hemmkonzentrationen]). Die erfindungsgemäßen Zusammensetzungen A1 bis A6 zeigen eine gute antibakterielle Aktivität, wobei die Zusammensetzung A6 die beste antibakterielle Aktivität zeigt, gefolgt von den Zusammensetzungen A4, A3, A2, A5 und A1 (in der Reihenfolge der antibakteriellen Wirksamkeit); die nichterfindungsgemäße Zusammensetzung A7 zeigt im Wesentlichen keine antibakterielle Aktivität.

Die antivirale Aktivität der Zusammensetzungen wird gegen IBV (Infektiöses Bronchitis-Virus, IBV Gray Stamm) in einem sogenannten MTT-Test in Vero-E6 (*African green monkey kidney cells* [Affennierenzellen]) getestet (Ermittlung der IC₅₀-Konzentrationen). Die erfindungsgemäßen Zusammensetzungen A1 bis A6 zeigen eine gute antivirale Aktivität (Anti-IBV-Eigenschaften), wobei die Zusammensetzung A6 die beste antivirale Aktivität zeigt, gefolgt von den Zusammensetzungen A4, A3, A2, A5 und A1 (in der Reihenfolge der antiviralen Wirksamkeit); die nichterfindungsgemäße Zusammensetzung A7 zeigt im Wesentlichen keine antivirale Aktivität.

### 3. Anwendungsbeobachtungen und klinische Untersuchungen

### Teil A

Die in dem vorstehenden Ausführungsbeispiel 2 b) beschriebenen erfindungsgemäßen Zusammensetzungen A1 bis A6 sowie die in dem vorstehenden Ausführungsbeispiel 2 c) beschriebene nichterfindungsgemäße Zusammensetzung A7 werden in klinischen Anwendungen (randomisierte placebokontrollierte Doppelblindstudien) getestet.

Je 12 Probanden eines aus 96 Patienten bestehenden Probandenkollektivs im Alter von 18 bis 77 Jahren (31 männlich, 58 weiblich) jeweils mit akuten viralen Rhinitiden ("Erkältungsschnupfen") werden während der Dauer ihrer Erkrankung 5 Tage lang mit den Zusammensetzungen A1 bis A7 behandelt, wobei die Zusammensetzungen A1 bis A7 mehrmals täglich als Spray topisch appliziert werden. Weitere 12 Probanden des Probandenkollektivs werden mit einer nichterfindungsgemäßen Zusammensetzung A8 therapiert, wobei die nichterfindungsgemäße Zusammensetzung A8 keine Wirkstoffe enthält (Placebokontrolle).

Als Zielvariablen (Zielparameter) werden die Bewertung der Nasenatmungsbehinderung, der Rhinorrhö (Fließschnupfen), der Rötung der Nasenschleimhaut, der Trockenheit der Nasenschleimhaut, der Borkenbildung, des Heilungsverlaufs und der Verträglichkeit dokumentiert.

Alle getesteten Zusammensetzungen zeigen eine ausgezeichnete Verträglichkeit. Bezüglichkeit der Beschleunigung des Heilungsverlaufs liefert die Zusammensetzung A6 das beste Ergebnis, gefolgt von den Zusammensetzungen A4, A3, A2, A5 und A1 (in der Reihenfolge der Wirksamkeit bzw. der Beschleunigung des Heilungsverlaufs); die nichterfindungsgemäße Zusammensetzung A7 und A8 führen im Wesentlichen zu keiner Beschleunigung des Heilungsverlaufs.

Bezüglich der übrigen Zielvariablen (Zielparameter), d. h. Bewertung der Nasenatmungsbehinderung, Bewertung der Rhinorrhö (Fließschnupfen) und Bewertung der Rötung der Nasenschleimhaut, sind die Bewertungen in Form von sogenannten Scores (Score-Werten) in der nachfolgenden Tabelle für die verschiedenen Zusammensetzungen wiedergegeben (0 = keine, 1 = geringe, 2 = mäßige, 3 = starke und 4 = sehr starke Ausprägung); ein höherer Score-Wert zeigt somit eine höhere Aktivität der Erkrankung bzw. eine schlechtere Wirksamkeit an.

| | Zusammensetzung | | | | | | | |
|---|---|---|---|---|---|---|---|---|
| | A1 | A2 | A3 | A4 | A5 | A6 | A7* | A8* |
| Nasenatm.-Behind. | | | | | | | | |
| Basiswert | 2,9±0,6 | 2,9±0,5 | 2,8±0,6 | 2,8±0,7 | 2,9±0,5 | 2,9±0,4 | 2,8±0,5 | 2,9±0,6 |
| 3 Tage | 1,9±0,8 | 1,8±0,6 | 1,7±0,6 | 1,6±0,5 | 1,9±0,6 | 1,4±0,5 | 2,5±0,9 | 2,7±0,9 |
| 5 Tage | 1,5±0,8 | 1,3±0,5 | 1,1±0,4 | 1,0±0,4 | 1,4±0,6 | 0,8±0,3 | 2,0±0,8 | 2,3±1,0 |
| | | | | | | | | |

| Rhinorrhö | | | | | | | | |
|---|---|---|---|---|---|---|---|---|
| Basiswert | 3,1±0,6 | 3,0±0,5 | 2,9±0,7 | 3,0±0,4 | 3,2±0,7 | 3,1±0,6 | 2,9±0,5 | 3,0±0,7 |
| 3 Tage | 1,7±0,7 | 1,4±0,5 | 1,3±0,4 | 1,2±0,3 | 1,6±0,5 | 1,0±0,3 | 2,6±0,8 | 2,7±1,0 |
| 5 Tage | 1,0±0,6 | 0,8±0,4 | 0,7±0,3 | 0,6±0,3 | 0,9±0,5 | 0,4±0,1 | 2,0±0,9 | 2,1±1,1 |
| | | | | | | | | |

| Rötung Nasenschleimhaut | | | | | | | | |
|---|---|---|---|---|---|---|---|---|
| Basiswert | 3,0±0,5 | 3,2±0,7 | 2,9±0,6 | 2,9±0,5 | 3,0±0,4 | 3,1±0,5 | 3,0±0,4 | 3,0±0,4 |
| 3 Tage | 1,7±0,8 | 1,5±0,6 | 1,4±0,5 | 1,3±0,4 | 1,6±0,7 | 1,1±0,4 | 2,0±0,7 | 2,7±1,1 |
| 5 Tage | 0,9±0,6 | 0,8±0,3 | 0,6±0,2 | 0,5±0,2 | 0,9±0,5 | 0,3±0,1 | 1,5±0,6 | 2,2±0,9 |
| 5 Tage | 1,3±0,7 | 1,0±0,4 | 0,9±0,4 | 0,7±0,3 | 1,1±0,5 | 0,5±0,2 | 1,6±0,9 | 2,1±1,0 |

| | | | | | | | | |
|---|---|---|---|---|---|---|---|---|
| * nicht erfindungsgemäß | | | | | | | | |

Insgesamt zeigen alle getesteten Zusammensetzungen A1 bis A6 nach der vorliegenden Erfindung eine ausgezeichnete Wirksamkeit bei der Behandlung akuter Rhinitiden. Bezüglichkeit der Wirksamkeit liefert die erfindungsgemäße Zusammensetzung A6 das beste Ergebnis, gefolgt von den erfindungsgemäßen Zusammensetzungen A4, A3, A2, A5 und A1 (in der Reihenfolge der Wirksamkeit). Die nichterfindungsgemäßen Zusammensetzungen A7 und A8 haben dagegen im Wesentlichen keine Wirksamkeit.

Weitergehende klinischen Untersuchungen sowie weiterführende randomisierte placebokontrollierte Doppelblindstudien bestätigen darüber hinaus einen Synergismus der Wirkungen der Wirkstoffe (d. h. Cineol und Dexpanthenol) der erfindungsgemäßen Zusammensetzungen, was bei der Behandlung von Rhinitiden zu einer klinisch deutlicheren bzw. signifikanteren Besserung führt, und zwar über das Ausmaß der Einzelwirkstoffe deutlich hinausgehend.

### Teil B

Weiterhin werden zwei weitere Zusammensetzungen, welche jeweils ein Dekongestivum enthalten, hergestellt und getestet:
Entsprechend der vorstehenden allgemeinen Herstellvorschrift werden zwei wässrige Zusammensetzungen ("Zusammensetzungen A9 und A10") hergestellt: Die erfindungsgemäße Zusammensetzung A9 enthält 0,650 Gew.-% 1,8-Cineol und 5,250 Gew.-% Dexpanthenol sowie darüber hinaus 0,050 Gew.-% Xylometazolinhydrochlorid als Dekongestivum, 1,500 Gew.-% Emulgator und 0,065 Gew.-% Konservierungs-/Desinfektionsmittel (jeweils bezogen auf die Zusammensetzung), wobei mit einem wie zuvor spezifizierten pH-Wertpuffersystem auf Phosphatbasis (0,853 Gew.-% Kaliumdihydrogenphosphat und 0,027 Gew.-% Natriummonohydrogenphosphat-Dodekahydrat, jeweils bezogen auf die Zusammensetzung) ein pH-Wert von etwa 5,5 eingestellt wird. Die nichterfindungsgemäße Zusammensetzung A10 entspricht der Zusammensetzung A9, jedoch mit der Abweichung, dass zum einen der Cineolanteil vollständig durch Wasser ersetzt ist (d. h. die nichterfindungsgemäße Zusammensetzung A10 enthält kein 1,8-Cineol) und zum anderen der Xylometazolinhydrochlorid-Anteil verdoppelt ist (d. h. der Xylometazolinhydrochlorid-Anteil beträgt 0,100 Gew.-%, bezogen auf die Zusammensetzung).

Die Zusammensetzungen A9 und A10 werden in klinischen Anwendungen (randomisierte placebokontrollierte Doppelblindstudien) getestet: Je 11 Probanden eines aus 22 Patienten bestehenden Probandenkollektivs im Alter von 19 bis 79 Jahren (10 männlich, 12 weiblich) jeweils mit akuten viralen Rhinitiden ("Erkältungsschnupfen") werden während der Dauer ihrer Erkrankung 5 Tage lang mit den Zusammensetzungen A9 und A10 behandelt, wobei die Zusammensetzungen mehrmals täglich als Spray topisch appliziert werden. Als Zielvariablen (Zielparameter) werden die Bewertung der Nasenatmungsbehinderung, der Rhinorrhö (Fließschnupfen), der Rötung der Nasenschleimhaut, der Trockenheit der Nasenschleimhaut, der Borkenbildung, des Heilungsverlaufs und der Verträglichkeit dokumentiert.

Bezüglich der Zielvariablen (Zielparameter) der Nasenatmungsbehinderung, welche die dekongestive Wirkung indiziert, werden bei der erfindungsgemäßen Zusammensetzung A9 nach 3 bzw. 5 Tagen Score-Werte von (1,0 ± 0,3) bzw. (0,5 ± 0,2) erreicht (Basiswert: 3,1 ± 0,6), während bei der nichterfindungsgemäßen Zusammensetzung A10 trotz doppelter Menge an Dekongestivum die Score-Werte nach 3 bzw. 5 Tagen (1,2 ± 0,4) bzw. (0,7 ± 0,3) betragen (Basiswert: 3,0 ± 0,4).

Dies zeigt, dass die Anwesenheit des Cineols überraschenderweise eine synergistische Wechselwirkung mit dem Dekongestivum bewirkt, so dass die Wirkung des Dekongestivums signifikant erhöht wird. Auf diese Weise kann die Menge an Dekongestivum bei gleichzeitiger Wirkungssteigerung verringert werden, was zu einer signifikanten Verbesserung des Nebenwirkungsprofils führt.

## Patentansprüche

1. Cineolhaltige Zusammensetzung,
wobei die Zusammensetzung in Kombination und jeweils in wirksamen, insbesondere pharmazeutisch wirksamen Mengen enthält:
(a) 1,8-Cineol, wobei das Cineol als Reinstoff vorliegt, wobei das Cineol eine Reinheit von mindestens 95 Gew.-%, bezogen auf das Cineol, aufweist und wobei das Cineol frei von anderen Terpenen ist und wobei die Zusammensetzung die Komponente (a), bezogen auf die Zusammensetzung, in relativen Mengen im Bereich von 0,001 bis 10 Gew.-% enthält;
und
(b)
(b1) Pantothenol, vorzugsweise Dexpanthenol (D-Pantothenol), oder dessen physiologisch unbedenkliche Ester und/oder
(b2) Pantothensäure oder deren physiologisch unbedenkliche Salze, wobei die Zusammensetzung die Komponente (b), bezogen auf die Zusammensetzung, in relativen Mengen im Bereich von 0,01 bis 10 Gew.-% enthält;
wobei die Zusammensetzung einen pH-Wert im Bereich von 5,0 bis 6,5 aufweist.

2. Zusammensetzung nach Anspruch 1,
wobei das Cineol eine Reinheit von mindestens 96 Gew.-%, bevorzugt mindestens 97 Gew.-%, besonders bevorzugt mindestens 98 Gew.-%, ganz besonders bevorzugt mindestens 99 Gew.-%, noch mehr bevorzugt mindestens 99,5 %, bezogen auf das Cineol, aufweist; und/oder
wobei die Zusammensetzung die Komponente (a), bezogen auf die Zusammensetzung, in relativen Mengen im Bereich von 0,002 bis 5 Gew.-%, vorzugsweise 0,01 bis 3 Gew.-%, bevorzugt 0,05 bis 3 Gew.-%, besonders bevorzugt 0,1 bis 2,5 Gew.-%, ganz besonders bevorzugt 0,2 bis 2 Gew.-%, noch mehr bevorzugt 0,3 bis 1 Gew.-%, am meisten bevorzugt 0,4 bis 0,8 Gew.-%, enthält.

3. Zusammensetzung nach Anspruch 1 oder 2,
wobei die Zusammensetzung als Komponente (b) Pantothenol, vorzugsweise Dexpanthenol (D-Pantothenol), oder dessen physiologisch unbedenkliche Ester enthält, vorzugweise Pantothenol, bevorzugt in Form von Dexpanthenol (D-Pantothenol); und/oder
wobei die Zusammensetzung die Komponente (b), bezogen auf die Zusammensetzung, in relativen Mengen im Bereich von 0,1 bis 8 Gew.-%, vorzugsweise 0,5 bis 7 Gew.-%, bevorzugt 1 bis 6,5 Gew.-%, besonders bevorzugt 2 bis 6 Gew.-%, ganz besonders bevorzugt 4 bis 6 Gew.-%, noch mehr bevorzugt 4,5 bis 5,5 Gew.-%, enthält); und/oder
wobei die Zusammensetzung die Komponenten (a) und (b) in einem Mengenverhältnis von Komponente (a) zu Komponente (b) im Bereich von 1 : 1 bis 1 : 500, insbesondere 1 : 1,25 bis 1 : 100, vorzugsweise 1 : 1,5 bis 1 : 75, bevorzugt 1 : 2 bis 1 : 50, besonders bevorzugt 1 : 2,5 bis 1 : 25, ganz besonders bevorzugt 1 : 2,75 bis 1 : 20, noch mehr bevorzugt 1 : 3 bis 1 : 10, enthält.

4. Zusammensetzung nach einem der vorangehenden Ansprüche,
wobei die Zusammensetzung außerdem als Komponente (c) mindestens einen Emulgator enthält;
insbesondere wobei die Zusammensetzung die Komponente (c) und/oder den Emulgator, bezogen auf die Zusammensetzung, in relativen Mengen im Bereich von 0,0001 bis 10 Gew.-%, insbesondere 0,001 bis 5 Gew.-%, vorzugsweise 0,005 bis 4 Gew.-%, bevorzugt 0,01 bis 3 Gew.-%, besonders bevorzugt 0,05 bis 2,5 Gew.-%, ganz besonders bevorzugt 0,1 bis 2 Gew.-%, noch mehr bevorzugt 0,2 bis 1,8 Gew.-%, am meisten bevorzugt 0,5 bis 1,5 Gew.-%, enthält; und/oder
insbesondere wobei die Komponente (c) und/oder der Emulgator ausgewählt ist aus der Gruppe von (i) Fettsäuren, Fettsäuresalzen, Fettsäureamiden und Fettsäureestern, insbesondere Fettsäureestern, (ii) gegebenenfalls (poly)ethoxylierten und/oder hydrierten Rizinusölen (Castorölen), (iii) organischen Carbonsäuren und deren Salzen und Estern, insbesondere Citronensäure und deren Salzen (Citraten), (iv) organischen Betainen, (v) organischen Ammoniumverbindungen, (vi) physiologisch unbedenklichen ionischen oder nichtionischen Tensiden, (vii) Phospholipiden, insbesondere Phosphatidylcholinen und Lecithinen, (viii) Poloxameren (Ethylenoxid/Propylenoxid-Blockcopolymeren), (ix) Estern von mehrwertigen Alkoholen (Polyalkoholen), insbesondere Glycerinestern, (x) Glykolen, insbesondere Polyalkylenglykolen, vorzugsweise Polyethylenglykolen, und deren (Poly-)Ethern und (Poly-)Estern, sowie Kombinationen aus zwei oder mehreren der vorgenannten Verbindungen, bevorzugt aus der Gruppe von (i) Fettsäuren, Fettsäuresalzen, Fettsäureamiden und Fettsäureestern, insbesondere Fettsäureestern, und (ii) gegebenenfalls (poly)ethoxylierten und/oder hydrierten Rizinusölen sowie deren Kombinationen.

5. Zusammensetzung nach einem der vorangehenden Ansprüche,
wobei die Zusammensetzung außerdem als Komponente (d) mindestens ein chemisches Puffersystem, insbesondere in Form von Puffersalz(en), enthält;
insbesondere wobei die Zusammensetzung die Komponente (d) und/oder das chemische Puffersystem, bezogen auf die Zusammensetzung und berechnet als Summe aller Bestandteile des chemischen Puffersystems, in relativen Mengen im Bereich von 0,001 bis 4 Gew.-%, insbesondere 0,01 bis 3 Gew.-%, vorzugsweise 0,05 bis 2 Gew.-%, bevorzugt 0,1 bis 1,5 Gew.-%, besonders bevorzugt 0,2 bis 1 Gew.-%, enthält; und/oder
insbesondere wobei die Komponente (d) und/oder das chemische Puffersystem zur Einstellung und/oder Konstanthaltung des pH-Werts der Zusammensetzung dient; und/oder
insbesondere wobei die Komponente (d) und/oder das chemische Puffersystem als ein Dihydrogenphosphat/Monohydrogenphosphat-Puffersystem ("H₂PO₄⁻/HPO₄²⁻-Puffer(system)" bzw. "Phosphatpuffer(system)"), insbesondere als ein Alkalidihydrogenphosphat/Alkalimonohydrogenphosphat-Puffersystem, vorliegt, bevorzugt mit einem Dihydrogenphosphat/Monohydrogenphosphat-Molverhältnis von größer 5 : 1, insbesondere im Bereich von 5 : 1 bis 110 : 1, besonders bevorzugt im Bereich von 6 : 1 bis 105 : 1, ganz besonders bevorzugt im Bereich von 7 : 1 bis 200 : 1, noch mehr bevorzugt im Bereich von 7 : 1 bis 100 : 1.

6. Zusammensetzung nach einem der vorangehenden Ansprüche,
wobei die Zusammensetzung einen pH-Wert im Bereich von 5,0 bis 6,2, bevorzugt im Bereich von 5,0 bis 6,0, noch mehr bevorzugt im Bereich von 5,1 bis 6,0, ganz besonders bevorzugt im Bereich von 5,2 bis 5,9, am meisten bevorzugt im Bereich von 5,3 bis 5,9, aufweist und/oder wobei der pH-Wert der Zusammensetzung Wert im Bereich von 4,5 bis 8,0, insbesondere im Bereich von 5,0 bis 6,5, vorzugsweise im Bereich von 5,0 bis 6,2, bevorzugt im Bereich von 5,0 bis 6,0, noch mehr bevorzugt im Bereich von 5,1 bis 6,0, ganz besonders bevorzugt im Bereich von 5,2 bis 5,9, am meisten bevorzugt im Bereich von 5,3 bis 5,9, eingestellt und/oder konstantgehalten wird, vorzugsweise mittels mindestens eines chemischen Puffersystems.

7. Zusammensetzung nach einem der vorangehenden Ansprüche,
wobei die Zusammensetzung außerdem als Komponente (e) mindestens ein Konservierungsmittel und/oder Desinfektionsmittel (Antiseptikum) enthält;
insbesondere wobei die Zusammensetzung die Komponente (e) und/oder das Konservierungsmittel und/oder Desinfektionsmittel (Antiseptikum), bezogen auf die Zusammensetzung, in relativen Mengen im Bereich von 0,001 bis 10 Gew.-%, insbesondere 0,005 bis 5 Gew.-%, vorzugsweise 0,01 bis 2 Gew.-%, bevorzugt 0,01 bis 1 Gew.-%, besonders bevorzugt 0,01 bis 0,5 Gew.-%, noch mehr bevorzugt 0,02 bis 0,1 Gew.-%, enthält; und/oder
insbesondere wobei das Konservierungsmittel und/oder Desinfektionsmittel ausgewählt ist aus der Gruppe von (i) Alkylbenzyldimethylammoniumchloriden, insbesondere C₈-C₁₈-Alkylbenzyldimethylammoniumchloriden, und Mischungen von verschiedenen Alkylbenzyldimethylammoniumchloriden, vorzugsweise Benzalkoniumchlorid, (ii) Polyhexanid (iii) Sorbinsäure und deren Salzen (Sorbaten), insbesondere Kaliumsorbat, (iv) Chlorhexidin, (v) para-Hydroxybenzoesäureestern und Mischungen von verschiedenen para-Hydroxybenzoesäureestern, vorzugsweise Nipa-Estern, sowie Kombinationen aus zwei oder mehreren der vorgenannten Verbindungen, bevorzugt aus der aus der Gruppe von (i) Alkylbenzyldimethylammoniumchloriden, insbesondere C₈-C₁₈-Alkylbenzyldimethylammoniumchloriden, und Mischungen von verschiedenen Alkylbenzyldimethylammoniumchloriden, vorzugsweise Benzalkoniumchlorid, (ii) Polyhexanid, (iii) Sorbinsäure und deren Salzen (Sorbaten), insbesondere Kaliumsorbat, sowie Kombinationen aus zwei oder mehreren der vorgenannten Verbindungen, besonders bevorzugt aus der Gruppe von Benzalkoniumchlorid, Polyhexanid und Sorbaten sowie deren Kombinationen.

8. Zusammensetzung nach einem der vorangehenden Ansprüche,
wobei die Zusammensetzung außerdem als Komponente (f) mindestens ein Verdickungsmittel enthält;
insbesondere wobei die Zusammensetzung die Komponente (f) und/oder das Verdickungsmittel, bezogen auf die Zusammensetzung, in relativen Mengen im Bereich von 0,0001 bis 10 Gew.-%, insbesondere 0,001 bis 8 Gew.-%, vorzugsweise 0,005 bis 5 Gew.-%, bevorzugt 0,01 bis 3 Gew.-%, besonders bevorzugt 0,05 bis 2 Gew.-%, noch mehr bevorzugt 0,1 bis 1 Gew.-%, enthält; und/oder
insbesondere wobei die Komponente (f) und/oder das Verdickungsmittel ausgewählt ist aus der Gruppe von (i) vorzugsweise sauren Glykosaminoglykanen oder deren physiologisch unbedenkliche Salzen oder Derivaten, insbesondere Hyaluronsäuren oder deren physiologisch unbedenklichen Salzen (Hyaluronaten), (ii) Polysacchariden und Hydrokolloiden, insbesondere Carrageenen (Carrageenanen) (iii) Cellulosen und Cellulosederivaten, insbesondere Celluloseestern und Celluloseethern, (iv) Alginsäuren und deren Salzen (Alginaten), (v) Poly(meth)acrylsäuren und Poly(meth)acrylaten, (vi) Polyalkylenglykolen, insbesondere Polyethylenglykolen, (vii) Xanthanen sowie Kombinationen aus zwei oder mehreren der vorgenannten Verbindungen, bevorzugt aus der aus der Gruppe von Hyaluronsäuren oder deren physiologisch unbedenklichen Salze (Hyaluronaten), Carrageenen (Carrageenanen) sowie deren Kombinationen.

9. Zusammensetzung nach einem der vorangehenden Ansprüche,
wobei die Zusammensetzung außerdem als Komponente (g) mindestens ein Ectoin oder Ectoinderivat, insbesondere ein Hydroxyectoin, enthält, insbesondere in relativen Mengen im Bereich von 0,0001 bis 10 Gew.-%, insbesondere 0,001 bis 5 Gew.-%, vorzugsweise 0,01 bis 2 Gew.-%, bezogen auf die Zusammensetzung; und/oder
wobei die Zusammensetzung außerdem als Komponente (h) mindestens ein vorzugsweise imidazolinbasiertes alpha-Sympathomimetikum oder dessen physiologisch unbedenkliche Salze enthält;
insbesondere wobei das vorzugsweise imidazolinbasierte alpha-Sympathomimetikum ausgewählt ist aus Xylometazolin oder Oxymetazolin, insbesondere in Form von deren physiologisch unbedenklichen Salzen, besonders bevorzugt in Form von deren Hydrochloridsalzen, und besonders bevorzugt Xylometazolinhydrochlorid ist; und/oder
insbesondere wobei die Zusammensetzung das vorzugsweise imidazolinbasierte alpha-Sympathomimetikum, bezogen auf die Zusammensetzung, in relativen Mengen im Bereich von 0,001 bis 2 Gew.-%, insbesondere 0,005 bis 1,5 Gew.-%, vorzugsweise 0,01 bis 1,2 Gew.-%, bevorzugt 0,02 bis 1,0 Gew.-%, besonders bevorzugt 0,03 bis 0,5 Gew.-%, ganz besonders bevorzugt 0,04 bis 0,2 Gew.-%, enthält; und/oder
wobei die Zusammensetzung außerdem Natriumchlorid enthält, insbesondere in relativen Mengen im Bereich von 0,001 bis 5 Gew.-%, insbesondere 0,01 bis 2 Gew.-%, vorzugsweise 0,1 bis 1 Gew.-%, bezogen auf die Zusammensetzung

10. Zusammensetzung nach einem der vorangehenden Ansprüche,
wobei die Zusammensetzung in Kombination und jeweils in wirksamen, insbesondere pharmazeutisch wirksamen Mengen enthält:
(a) Cineol, vorzugsweise 1,8-Cineol, in relativen Mengen im Bereich von 0,001 bis 10 Gew.-%, insbesondere 0,002 bis 5 Gew.-%, vorzugsweise 0,01 bis 3 Gew.-%, bevorzugt 0,05 bis 3 Gew.-%, besonders bevorzugt 0,1 bis 2,5 Gew.-%, ganz besonders bevorzugt 0,2 bis 2 Gew.-%, noch mehr bevorzugt 0,3 bis 1 Gew.-%, am meisten bevorzugt 0,4 bis 0,8 Gew.-%;
(b) Pantothenol, vorzugsweise Dexpanthenol (D-Pantothenol), oder dessen physiologisch unbedenkliche Ester, in relativen Mengen im Bereich von 0,01 bis 10 Gew.-%, insbesondere 0,1 bis 8 Gew.-%, vorzugsweise 0,5 bis 7 Gew.-%, bevorzugt 1 bis 6,5 Gew.-%, besonders bevorzugt 2 bis 6 Gew.-%, ganz besonders bevorzugt 4 bis 6 Gew.-%, noch mehr bevorzugt 4,5 bis 5,5 Gew.-%;
(c) gegebenenfalls mindestens einen Emulgator, insbesondere in relativen Mengen im Bereich von 0,0001 bis 5 Gew.-%, insbesondere 0,001 bis 2,5 Gew.-%, vorzugsweise 0,005 bis 2 Gew.-%, bevorzugt 0,01 bis 1,5 Gew.-%, besonders bevorzugt 0,01 bis 1 Gew.-%, ganz besonders bevorzugt 0,01 bis 0,5 Gew.-%, noch mehr bevorzugt 0,02 bis 0,08 Gew.-%;
(d) gegebenenfalls mindestens ein chemisches Puffersystem, insbesondere in Form von Puffersalz(en), insbesondere in relativen Mengen, berechnet als Summe aller Bestandteile des chemischen Puffersystems, im Bereich von 0,001 bis 4 Gew.-%, insbesondere 0,01 bis 3 Gew.-%, vorzugsweise 0,05 bis 2 Gew.-%, bevorzugt 0,1 bis 1,5 Gew.-%, besonders bevorzugt 0,2 bis 1 Gew.-%;
(e) gegebenenfalls mindestens ein Konservierungsmittel und/oder Desinfektionsmittel (Antiseptikum), insbesondere in relativen Mengen im Bereich von 0,001 bis 10 Gew.-%, insbesondere 0,005 bis 5 Gew.-%, vorzugsweise 0,01 bis 2 Gew.-%, bevorzugt 0,01 bis 1 Gew.-%, besonders bevorzugt 0,01 bis 0,5 Gew.-%, noch mehr bevorzugt 0,02 bis 0,1 Gew.-%;
(f) gegebenenfalls mindestens ein Verdickungsmittel, insbesondere in relativen Mengen im Bereich von 0,0001 bis 10 Gew.-%, insbesondere 0,001 bis 8 Gew.-%, vorzugsweise 0,005 bis 5 Gew.-%, bevorzugt 0,01 bis 3 Gew.-%, besonders bevorzugt 0,05 bis 2 Gew.-%, noch mehr bevorzugt 0,1 bis 1 Gew.-%;
(g) gegebenenfalls mindestens ein Ectoin oder Ectoinderivat, insbesondere ein Hydroxyectoin, insbesondere in relativen Mengen im Bereich von 0,0001 bis 10 Gew.-%, insbesondere 0,001 bis 5 Gew.-%, vorzugsweise 0,01 bis 2 Gew.-%;
(h) gegebenenfalls mindestens ein vorzugsweise imidazolinbasiertes alpha-Sympathomimetikum oder dessen physiologisch unbedenkliche Salze, insbesondere in relativen Mengen im Bereich von 0,001 bis 2 Gew.-%, insbesondere 0,005 bis 1,5 Gew.-%, vorzugsweise 0,01 bis 1,2 Gew.-%, bevorzugt 0,02 bis 1,0 Gew.-%, besonders bevorzugt 0,03 bis 0,5 Gew.-%, ganz besonders bevorzugt 0,04 bis 0,2 Gew.-%
(i) Natriumchlorid, insbesondere in relativen Mengen im Bereich von 0,001 bis 5 Gew.-%, insbesondere 0,01 bis 2 Gew.-%, vorzugsweise 0,1 bis 1 Gew.-%;
wobei alle vorgenannten relativen Mengenangaben auf die Zusammensetzung bezogen sind.

11. Zusammensetzung nach einem der vorangehenden Ansprüche,
wobei die Zusammensetzung als wässrige Zusammensetzung vorliegt und/oder wobei die Zusammensetzung wässrig basiert ist und/oder wässrig formuliert vorliegt, insbesondere in Form einer wässrigen Lösung oder wässrigen Solubilisierung.

12. Zusammensetzung nach einem der vorangehenden Ansprüche,
wobei die Zusammensetzung bei einer Temperatur von 20 °C in Abwesenheit eines Verdickungsmittels eine dynamische Viskosität im Bereich von 1,001 bis 2 mPas, insbesondere 1,01 bis 1,9 mPas, vorzugsweise 1,05 bis 1,8 mPas, besonders bevorzugt 1,1 bis 1,5 mPas, aufweist (insbesondere bestimmt gemäß der Methode nach Ph. Eur. 7. Ausgabe, Grundwerk 2011, Abschnitt 2.2.9 Kapillarviskosimeter); oder aber
wobei die Zusammensetzung bei einer Temperatur von 20 °C in Anwesenheit eines Verdickungsmittels eine dynamische Viskosität von mindestens 1,5 mPas, insbesondere im Bereich von 1,5 bis 10⁵ mPas, vorzugsweise 1,6 bis 10⁴ mPas, besonders bevorzugt 1,7 bis 10³ mPas, noch mehr bevorzugt 2 bis 100 mPas, aufweist (insbesondere bestimmt gemäß der Methode nach Ph. Eur. 7. Ausgabe, Grundwerk 2011, Abschnitt 2.2.9 Kapillarviskosimeter).

13. Zusammensetzung nach einem der vorangehenden Ansprüche,
wobei die Zusammensetzung mindestens einen weiteren Inhaltsstoff, insbesondere Hilfsstoff und/oder Additiv, aufweist, insbesondere ausgewählt aus der Gruppe von Verarbeitungshilfsstoffen, Stabilisatoren, Emulgatoren, Antioxidantien, Feuchthaltemitteln, Verdickungsmitteln, Antiseptika, Farbstoffen, Aromastoffen, Riechstoffen, Duftstoffen, Streckmitteln, Bindemitteln, Netzmitteln, Vitaminen, Spurenelementen, Mineralstoffen, Mikronährstoffen und/oder ätherischen Ölen sowie deren Kombinationen.

14. Zusammensetzung nach einem der vorangehenden Ansprüche
zur Verwendung bei der prophylaktischen und/oder kurativen topischen Behandlung von *Rhinitis acuta;* oder
zur Verwendung bei der prophylaktischen und/oder kurativen topischen Behandlung Behandlung von Rhinitiden aller Art, insbesondere von *Rhinitis acuta, Rhinitis allergica, Rhinitis atrophicans, Rhinitis hyperplastica* oder *hypertrophicans, Rhinitis mutilans, Rhinitis nervosa* oder *vasomotorica,* umweltbedingter Rhinitis oder *Rhinitis pseudomembranacea,* vorzugsweise von *Rhinitis acuta.*

15. Applikationsvorrichtung, insbesondere für die topische, insbesondere nasale, vorzugsweise intranasale Applikation, bevorzugt in Form eines Behältnisses mit Tropf- oder Sprüheinrichtung, enthaltend eine Zusammensetzung nach einem der vorangehenden Ansprüche.

## Claims

1. A cineole-containing composition,
wherein the composition contains in combination and in effective, in particular pharmaceutically effective, amounts in each case:
(a) 1,8-cineole, wherein the cineole is present as a pure substance, wherein the cineole has a purity of at least 95% by weight, relative to the cineole, and wherein the cineole is free of other terpenes and wherein the composition contains the component (a) in relative amounts in the range of 0.001 to 10% by weight relative to the composition;
and
(b)
(b1) pantothenol, preferably dexpanthenol (D-pantothenol), or its physiologically acceptable esters, and/or
(b2) pantothenic acid or its physiologically acceptable salts,
wherein the composition contains the component (b) in relative amounts in the range of 0.01 to 10% by weight relative to the composition;
wherein the composition has a pH value in the range of 5.0 to 6.5.

2. The composition according to claim 1,
wherein the cineole has a purity of at least 96% by weight, preferably at least 97% by weight, particularly preferably at least 98% by weight, very particularly preferably at least 99% by weight, even more preferably at least 99.5%, relative to the cineole; and/or
wherein the composition contains the component (a) in relative amounts in the range of 0.002 to 5% by weight relative to the composition, preferably 0.01 to 3% by weight, preferably 0.05 to 3% by weight, particularly preferably 0.1 to 2.5% by weight, very particularly preferably 0.2 to 2% by weight, even more preferably 0.3 to 1% by weight, most preferably 0.4 to 0.8% by weight.

3. The composition according to claim 1 or 2,
wherein the composition contains, as the component (b), pantothenol, preferably dexpanthenol (D-pantothenol), or its physiologically acceptable ester, preferably pantothenol, preferably in the form of dexpanthenol (D-pantothenol); and/or
wherein the composition contains the component (b) in relative amounts in the range of 0.1 to 8% by weight relative to the composition, preferably 0.5 to 7% by weight, preferably 1 to 6.5% by weight, particularly preferably 2 to 6% by weight, very particularly preferably 4 to 6% by weight, even more preferably 4.5 to 5.5% by weight; and/or
wherein the composition contains the components (a) and (b) in an amount ratio of component (a) to component (b) in the range of 1:1 to 1:500, in particular 1:1.25 to 1:100, preferably 1:1.5 to 1:75, preferably 1:2 to 1:50, particularly preferably 1:2.5 to 1:25, very particularly preferably 1:2.75 to 1:20, even more preferably 1:3 to 1:10.

4. The composition according to any one of the preceding claims,
wherein the composition also contains, as the component (c), at least one emulsifier;
In particular wherein the composition contains the component (c) and/or the emulsifier in relative amounts in the range of 0.0001 to 10% by weight relative to the composition, particularly 0.001 to 5% by weight, preferably 0.005 to 4% by weight, preferably 0.01 to 3% by weight, particularly preferably 0.05 to 2.5% by weight, very particularly preferably 0.1 to 2% by weight, even more preferably 0.2 to 1.8% by weight, most preferably 0.5 to 1.5% by weight; and/or
in particular wherein the component (c) and/or the emulsifier is selected from the group of (i) fatty acids, fatty acid salts, fatty acid amides, and fatty acid esters, in particular fatty acid esters, (ii) optionally (poly)ethoxylated and/or hydrogenated castor oils, (iii) organic carboxylic acids and their salts and esters, in particular citric acid and its salts (citrates), (iv) organic betaines, (v) organic ammonium compounds, (vi) physiologically acceptable ionic or nonionic surfactants, (vii) phospholipids, in particular phosphatidylcholines and lecithins, (viii) poloxamers (ethylene oxide/propylene oxide block copolymers), (ix) esters of polyhydric alcohols (polyalcohols), in particular glycerol esters, (x) glycols, in particular polyalkylene glycols, preferably polyethylene glycols, and their (poly)ethers and (poly)esters, and combinations of two or more of the aforementioned compounds, preferably from the group of (i) fatty acids, fatty acid salts, fatty acid amides, and fatty acid esters, in particular fatty acid esters, and (ii) optionally (poly)ethoxylated and/or hydrogenated castor oils and combinations thereof.

5. The composition according to any one of the preceding claims,
wherein the composition also contains, as the component (d), at least one chemical buffer system, in particular in the form of buffering salt(s);
in particular wherein the composition contains the component (d) and/or the chemical buffer system, in relative amounts in the range of 0.001 to 4% by weight in relation to the composition and calculated as a sum of all constituents of the chemical buffer system, in particular 0.01 to 3% by weight, preferably 0.05 to 2% by weight, more preferably 0.1 to 1.5% by weight, particularly preferably 0.2 to 1% by weight; and/or
in particular wherein the component (d) and/or the chemical buffer system is used to adjust and/or keep constant the pH value of the composition, and/or
In particular wherein the component (d) and/or the chemical buffer system exists as a dihydrogen phosphate/monohydrogen phosphate buffer system ("H₂PO₄⁻/HPO₄²⁻ buffer (system)" or "phosphate buffer (system)"), in particular as an alkali dihydrogen phosphate/alkali monohydrogen phosphate buffer system, preferably with a dihydrogen phosphate/monohydrogen phosphate molar ratio greater than 5:1, in particular in the range of 5:1 to 110:1, particularly preferably in the range of 6:1 to 105:1, more particularly preferably in the range of 7:1 to 200:1, even more preferably in the range of 7:1 to 100:1.

6. The composition according to any one of the preceding claims,
wherein the composition has a pH value in the range of 5.0 to 6.2, preferably in the range of 5.0 to 6.0, more preferably in the range of 5.1 to 6.0, even more preferably in the range of 5.2 to 5.9, most preferably in the range of 5.3 to 5.9, and/or wherein the pH value of the composition is set to or kept constant at a value in the range of 4.5 to 8.0, in particular in the range of 5.0 to 6.5, preferably in the range of 5.0 to 6.2, more preferably in the range of 5.0 to 6.0, even more preferably in the range of 5.1 to 6.0, very particularly preferably in the range of 5.2 to 5.9, most preferably in the range of 5.3 to 5.9, preferably by means of at least one chemical buffer system.

7. The composition according to any one of the preceding claims,
wherein the composition also contains, as the component (e), at least one preservative and/or disinfectant (antiseptic);
in particular wherein the composition contains the component (e) and/or the preservative and/or disinfectant (antiseptic) in relative amounts in the range of 0.001 to 10% by weight relative to the composition, in particular 0.005 to 5% by weight, preferably 0.01 to 2% by weight, preferably 0.01 to 1% by weight, particularly preferably 0.01 to 0.5% by weight, more preferably 0.02 to 0.1% by weight; and/or
in particular wherein the preservative and/or disinfectant is selected from the group of (i) alkylbenzyldimethylammonium chlorides, in particular C₈-C₁₈-alkylbenzyldimethylammonium chlorides, and mixtures of various alkylbenzyldimethylammonium chlorides, preferably benzalkonium chloride, (ii) polyhexanide (iii) sorbic acid and salts thereof (sorbates), in particular potassium sorbate, (iv) chlorhexidine, (v) para-hydroxybenzoic acid esters and mixtures of different para-hydroxybenzoic acid esters, preferably Nipa esters, and combinations of two or more of the aforementioned compounds, preferably from the group of (i) alkylbenzyldimethylammonium chlorides, in particular C₈-C₁₈-alkylbenzyldimethylammonium chlorides, and mixtures of various alkylbenzyldimethylammonium chlorides, preferably benzalkonium chloride, (ii) polyhexanide, (iii) sorbic acid and its salts (sorbates), in particular potassium sorbate, and combinations of two or more of the aforementioned compounds, more preferably from the group of benzalkonium chloride, polyhexanide and sorbates and combinations thereof.

8. The composition according to any one of the preceding claims,
wherein the composition also contains, as the component (f), at least one thickening agent;
in particular wherein the composition contains the component (f) and/or the thickening agent in relative amounts in the range of 0.0001 to 10% by weight relative to the composition, in particular 0.001 to 8% by weight, preferably 0.005 to 5% by weight, preferably 0.01 to 3% by weight, particularly preferably 0.05 to 2% by weight, more preferably 0.1 to 1% by weight; and or
in particular wherein the component (f) and/or the thickening agent is selected from the group of (i) preferably acid glycosaminoglycans or their physiologically acceptable salts or derivatives, in particular hyaluronic acids or their physiologically acceptable salts (hyaluronates), (ii) polysaccharides and hydrocolloids, in particular carrageenans (carrageenans) (iii) celluloses and cellulose derivatives, in particular cellulose esters and cellulose ethers, (iv) alginic acids and their salts (alginates), (v) poly(meth)acrylic acids and poly(meth)acrylates, (vi) polyalkylene glycols, in particular polyethylene glycols , (vii) xanthans and combinations of two or more of the aforementioned compounds, preferably from the group of hyaluronic acids or their physiologically acceptable salts (hyaluronates), carrageenans (carrageenans) and combinations thereof.

9. The composition according to any one of the preceding claims,
wherein the composition also contains, as the component (g), at least one ectoine or ectoine derivative, in particular a hydroxyectoine, in particular in relative amounts in the range of 0.0001 to 10% by weight, in particular 0.001 to 5% by weight, preferably 0.01 to 2% by weight, relative to the composition; and/or
wherein the composition also contains, as the component (h), at least one preferably imidazoline-based alpha-sympathomimetic drug or its physiologically acceptable salts;
in particular wherein the preferably imidazoline-based alpha-sympathomimetic is selected from xylometazoline or oxymetazoline, in particular in the form of their physiologically acceptable salts, more preferably in the form of their hydrochloride salts, and most preferably xylometazoline hydrochloride; and/or
in particular wherein the composition contains the preferably imidazoline-based alpha-sympathomimetic in relative amounts in the range of 0.001 to 2% by weight relative to the composition, in particular 0.005 to 1.5% by weight, preferably 0.01 to 1.2% by weight, preferably 0.02 to 1.0% by weight, particularly preferably 0.03 to 0.5% by weight, very particularly preferably 0.04 to 0.2% by weight; and/or
wherein the composition further contains sodium chloride, in particular in relative amounts in the range of 0.001 to 5% by weight, in particular 0.01 to 2% by weight, preferably 0.1 to 1% by weight, relative to the composition.

10. The composition according to any one of the preceding claims,
wherein the composition contains in combination and in effective, in particular pharmaceutically effective, amounts in each case:
(a) Cineole, preferably 1,8-cineole, in relative amounts in the range of 0.001 to 10% by weight, in particular 0.002 to 5% by weight, preferably 0.01 to 3% by weight, preferably 0.05 to 3% by weight, particularly preferably 0.1 to 2.5% by weight, very particularly preferably 0.2 to 2% by weight, even more preferably 0.3 to 1% by weight, most preferably 0.4 to 0.8% by weight;
(b) pantothenol, preferably dexpanthenol (D-pantothenol), or its physiologically acceptable ester, in relative amounts in the range of 0.01 to 10% by weight, in particular 0.1 to 8% by weight, preferably 0.5 to 7% by weight, preferably 1 to 6.5% by weight, particularly preferably 2 to 6% by weight, very particularly preferably 4 to 6% by weight, even more preferably 4.5 to 5.5% by weight;
(c) wherein the composition contains at least one emulsifier, in particular in relative amounts in the range of 0.0001 to 5% by weight, in particular 0.001 to 2.5% by weight, preferably 0.005 to 2% by weight, preferably 0.01 to 1.5% by weight, particularly preferably 0.01 to 1% by weight, very particularly preferably 0.01 to 0.5% by weight, even more preferably 0.02 to 0.08% by weight;
(d) optionally at least one chemical buffer system, in particular in the form of buffer salt(s), in particular in relative amounts, calculated as the sum of all constituents of the chemical buffer system, in the range of 0.001 to 4% by weight, in particular 0.01 to 3% by weight, preferably 0.05 to 2% by weight, preferably 0.1 to 1.5% by weight, particularly preferably 0.2 to 1% by weight;
(e) optionally at least one preservative and/or disinfectant (antiseptic), in particular in relative amounts in the range of 0.001 to 10% by weight, in particular 0.005 to 5% by weight, preferably 0.01 to 2% by weight, preferably 0.01 to 1% by weight, more preferably 0.01 to 0.5% by weight, even more preferably 0.02 to 0.1% by weight;
(f) optionally at least one thickening agent, in particular in relative amounts in the range of 0.0001 to 10% by weight, in particular 0.001 to 8% by weight, preferably 0.005 to 5% by weight, preferably 0.01 to 3% by weight, more preferably 0.05 to 2% by weight, even more preferably 0.1 to 1% by weight;
(g) optionally at least one ectoine or ectoine derivative, in particular a hydroxyectoine, in particular in relative amounts in the range of 0.0001 to 10% by weight, in particular 0.001 to 5% by weight, preferably 0.01 to 2% by weight;
(h) optionally at least one preferably imidazoline-based alpha-sympathomimetic or its physiologically acceptable salts, in particular in relative amounts in the range of 0.001 to 2% by weight, in particular 0.005 to 1.5% by weight, preferably 0.01 to 1.2% by weight, preferably 0.02 to 1.0% by weight, particularly preferably 0.03 to 0.5% by weight, most preferably 0.04 to 0.2% by weight;
(i) sodium chloride, in particular in relative amounts in the range of 0.001 to 5% by weight, in particular 0.01 to 2% by weight, preferably 0.1 to 1% by weight;
wherein all of the aforementioned relative amounts are relative to the composition.

11. The composition according to any one of the preceding claims,
wherein the composition is present as an aqueous composition and/or wherein the composition is aqueous-based and/or has an aqueous formulation, in particular in the form of an aqueous solution or aqueous solubilisation.

12. The composition according to any one of the preceding claims,
wherein the composition, at a temperature of 20°C, in the absence of a thickening agent, has a dynamic viscosity in the range of 1.001 to 2 mPas, particularly 1.01 to 1.9 mPas, preferably 1.05 to 1.8 mPas, particularly preferably 1.1 to 1.5 mPas (in particular, determined by the method according to Ph. Eur. 7th edition, Main Volume 2011, section 2.2.9 Capillary Viscometer); or else
wherein the composition, at a temperature of 20°C, in the presence of a thickening agent, has a dynamic viscosity of at least 1.5 mPas, particularly in the range of 1.5 to 10⁵ mPas, preferably 1.6 to 10⁴ mPas, particularly preferably 1.7 to 10³ mPas, even more preferably 2 to 100 mPas, (in particular, determined by the method according to Ph. Eur. 7th edition, Main Volume 2011, section 2.2.9 Capillary Viscometer).

13. The composition according to any one of the preceding claims,
wherein the composition comprises at least one further ingredient, in particular an adjuvant and/or additive, in particular selected from the group of processing aids, stabilisers, emulsifiers, antioxidants, humectants, thickening agents, antiseptics, dyes, flavourings, fragrances, perfumes, extenders, binders, wetting agents, vitamins, trace elements, minerals, micronutrients and/or essential oils, and combinations thereof.

14. The composition according to any one of the preceding claims
for use in the prophylactic and/or curative topical treatment of *rhinitis acuta;* or
for use in prophylactic and/or curative topical treatment of rhinitis of all kinds, in particular *rhinitis acuta, rhinitis allergica, rhinitis atrophicans, rhinitis hyperplastica or hypertrophicans, rhinitis mutilans, rhinitis nervosa or vasomotorica,* environmental rhinitis or *rhinitis pseudomembranacea,* preferably of *rhinitis acuta.*

15. An application device, in particular for topical, in particular nasal, preferably intranasal, application, more preferably in the form of a container having a dropper or spraying means, containing a composition according to any one of the preceding claims.

## Revendications

1. Composition contenant du cinéol,
la composition contenant en association et pour chacun en des quantités efficaces, en particulier pharmaceutiquement efficaces :
(a) du 1,8-cinéol, le cinéol étant présent sous forme de substance pure, le cinéol ayant une pureté d'au moins 95 % en poids, par rapport au cinéol, et le cinéol étant exempt d'autres terpènes et la composition contenant le composant (a) par rapport à la composition, en quantités relatives allant de 0,001 à 10 % en poids ;
et
(b)
(b1) du pantothénol, de préférence du dexpanthénol (D-pantothénol), ou ses esters physiologiquement acceptables et / ou
(b2) de l'acide pantothénique ou ses sels physiologiquement acceptables,
la composition contenant le composant (b), par rapport à la composition, en quantités relatives allant de 0,01 à 10 % en poids ;
la composition ayant un pH situé dans la plage allant de 5,0 à 6,5.

2. Composition selon la revendication 1,
dans laquelle le cinéol a une pureté d'au moins 96 % en poids, de préférence d'au moins 97 % en poids, de manière particulièrement préférée d'au moins 98 % en poids, de manière tout particulièrement préférée d'au moins 99 % en poids, de manière encore plus préférée d'au moins 99,5 %, par rapport au cinéol ; et / ou
la composition contenant le composant (a), par rapport à la composition, en des quantités relatives situées dans la plage allant de 0,002 à 5 % en poids, de préférence de 0,01 à 3 % en poids, de manière plus préférée de 0,05 à 3 % en poids, de manière particulièrement préférée de 0,1 à 2,5 % en poids, de manière tout particulièrement préférée de 0,2 à 2 % en poids, de manière encore plus préférée de 0,3 à 1 % en poids, idéalement de 0,4 à 0,8 % en poids.

3. Composition selon la revendication 1 ou 2,
la composition contenant en tant que composant (b) du pantothénol, de préférence du dexpanthénol (D-pantothénol), ou ses esters physiologiquement acceptables, de préférence du pantothénol, de préférence sous forme de dexpanthénol (D-pantothénol); et / ou
la composition contenant le composant (b), par rapport à la composition, en des quantités relatives situées dans la plage allant de 0,1 à 8 % en poids, de préférence de 0,5 à 7 % en poids, de manière plus préférée de 1 à 6,5 % en poids, de manière particulièrement préférée de 2 à 6 % en poids, de manière encore plus préférée de 4 à 6 % en poids, idéalement de 4,5 à 5,5 % en poids; et / ou
la composition contenant les composants (a) et (b) en un rapport pondéral du composant (a) au composant (b) situé dans la plage allant de 1: 1 à 1: 500, en particulier de 1: 1,25 à 1: 100, de préférence de 1: 1,5 à 1: 75, préférentiellement de 1: 2 à 1: 50, préférablement de 1: 2,5 à 1: 25,
de manière particulièrement préférée de 1: 2,75 à 1: 20, encore plus préférablement de 1: 3 à 1: 10.

4. Composition selon une quelconque des revendications précédentes,
la composition contenant en outre au moins un émulsifiant en tant que composant (c) ;
en particulier la composition contenant le composant (c) et / ou l'émulsifiant, par rapport à la composition, en des quantités relatives situées dans la plage allant de 0,0001 à 10 % en poids, en particulier de 0,001 à 5 % en poids, de préférence de 0,005 à 4 % en poids, de manière plus préférée de 0,01 à 3 % en poids, de manière particulièrement préférée de 0,05 à 2,5 % en poids, de manière tout particulièrement préférée de 0,1 à 2 % en poids, de manière encore plus préférée de 0,2 à 1,8 % en poids, idéalement de 0,5 à 1,5 % en poids ; et / ou
en particulier dans laquelle le composant (c) et / ou l'émulsifiant est choisi dans le groupe constitué par (i) des acides gras, des sels d'acides gras, des amides d'acides gras et des esters d'acides gras, en particulier des esters d'acides gras, (ii) des huiles de ricin le cas échéant (poly)éthoxylées et / ou hydrogénées, (iii) des acides carboxyliques organiques et leurs sels et esters, en particulier l'acide citrique et ses sels (citrates), (iv) des bétaïnes organiques, (v) des composés organiques de l'ammonium, (vi) des tensioactifs ioniques ou non ioniques physiologiquement acceptables, (vii) des phospholipides, en particulier des phosphatidylcholines et des lécithines (viii) des poloxamères (copolymères à blocs oxyde d'éthylène / oxyde de propylène), (ix) des esters d'alcools polyhydroxylés (polyalcools), en particulier des esters de glycérol, (x) des glycols, en particulier des polyalkylèneglycols, de préférence des polyéthylèneglycols, et leurs (poly)éthers et (poly)esters, ainsi que les combinaisons de deux ou plusieurs des composés susmentionnés, de préférence des groupes (i) des acides gras, des sels d'acides gras, des amides d'acides gras et des esters d'acides gras, en particulier des esters d'acides gras, et (ii) des huiles de ricin le cas échéant (poly)éthoxylées et / ou hydrogénées et leurs combinaisons.

5. Composition selon une quelconque des revendications précédentes,
la composition contenant en outre en tant que composant (d), au moins un système tampon chimique, notamment sous forme de sel(s) tampon(s) ;
en particulier, la composition contenant le composant (d) et / ou le système tampon chimique, par rapport à la composition et exprimées par la somme de tous les composants du système tampon chimique, en des quantités relatives situées dans la plage allant de 0,001 à 4 % en poids, en particulier de 0,01 à 3 % en poids, de préférence de 0,05 à 2 % en poids, préférablement de 0,1 à 1,5 % en poids, de manière particulièrement préférée de 0,2 à 1 % en poids, et / ou
en particulier, le composant (d) et / ou le système tampon chimique servant à ajuster et/ou à maintenir constant le pH de la composition, et / ou
en particulier, le composant (d) et / ou le système tampon chimique étant présent sous la forme d'un système tampon dihydrogénophosphate/monohydrogénophosphate (« (système) tampon ou « (système) tampon phosphate» H₂PO₄⁻/HPO₄²⁻ »), en particulier sous la forme d'un système tampon dihydrogénophosphate de métal alcalin/monohydrogénophosphate de métal alcalin, de préférence avec un rapport molaire dihydrogénophosphate/monohydrogénophosphate supérieur à 5: 1, en particulier dans la plage de 5: 1 à 110: 1, de manière particulièrement préférée dans la plage allant de 6: 1 à 105: 1, de manière particulièrement préférée dans la plage allant de 7: 1 à 200: 1, de manière encore plus préférée dans la plage allant de 7: 1 à 100: 1.

6. Composition selon une quelconque des revendications précédentes,
la composition présentant un pH dans la plage allant de 5,0 à 6,2, de préférence dans la plage allant de 5,0 à 6,0, préférablement dans la plage allant de 5,1 à 6,0, plus préférablement dans la plage allant de 5,2 à 5,9, encore plus préférablement dans la plage allant de 5,3 à 5,9, et / ou le pH de la composition étant ajusté et/ou maintenu constant à une valeur située dans la plage allant de 4,5 à 8,0, en particulier dans la plage allant de 5,0 à 6,5, de préférence dans la plage allant de 5,0 à 6,2, préférablement dans la plage allant de 5,0 à 6,0, plus préférablement dans la plage allant de 5,1 à 6,0, encore plus préférablement dans la plage allant de 5,2 à 5,9, le plus préférablement dans la plage allant de 5,3 à 5,9, de préférence au moyen d'au moins un système tampon chimique.

7. Composition selon une quelconque des revendications précédentes,
la composition contenant en outre en tant que composant (e) au moins un conservateur et / ou désinfectant (antiseptique) ;
en particulier la composition contenant le composant (e) et / ou le conservateur et / ou le désinfectant (antiseptique), par rapport à la composition, en des quantités relatives situées dans la plage allant de 0,001 à 10 % en poids, en particulier de 0,005 à 5 % en poids, de préférence de 0,01 à 2 % en poids, de manière plus préférée de 0,01 à 1 % en poids, de manière particulièrement préférée de 0,01 à 0,5 % en poids, de manière davantage préférée de 0,02 à 0,1 % en poids ; et / ou
en particulier, dans laquelle le conservateur et / ou le désinfectant est choisi dans le groupe (i) des chlorures d'alkylbenzyldiméthylammonium, en particulier des chlorures d'(alkyle en C₈ à C₁₈) benzyldiméthylammonium et des mélanges de différents chlorures d'alkylbenzyldiméthylammonium, de préférence le chlorure de benzalkonium, (ii) du polyhexanide, (iii) de l'acide sorbique et ses sels (sorbates), en particulier le sorbate de potassium, (iv) de la chlorhexidine, (v) des esters d'acide para-hydroxybenzoïque et des mélanges de différents esters d'acide para-hydroxybenzoïque, de préférence les esters de type Nipa, et des combinaisons de deux ou plusieurs des composés susmentionnés, de préférence du groupe (i) des chlorures d'alkylbenzyldiméthylammonium , en particulier des chlorures d'(alkyle en C₈ à C₁₈) benzyldiméthylammonium, et des mélanges de divers chlorures d'alkylbenzyldiméthylammonium, de préférence le chlorure de benzalkonium, (ii) du polyhexanide, (iii) de l'acide sorbique et ses sels (sorbates), en particulier le sorbate de potassium, et des combinaisons de deux ou plusieurs des composés susmentionnés, plus préférablement du groupe du chlorure de benzalkonium, du polyhexanide et des sorbates et leurs combinaisons.

8. Composition selon une quelconque des revendications précédentes,
la composition contenant en outre en tant que composant (f) au moins un épaississant ;
en particulier la composition contenant le composant (f) et / ou l'agent épaississant, par rapport à la composition, en des quantités relatives situées dans la plage allant de 0,0001 à 10 % en poids, en particulier de 0,001 à 8 % en poids, de préférence de 0,005 à 5 % en poids , de préférence de 0,01 à 3 % en poids, de manière particulièrement préférée de 0,05 à 2 % en poids, plus préférablement 0,1 à 1 % en poids ; et / ou
en particulier dans laquelle le composant (f) et / ou l'épaississant sont choisis dans le groupe (i) des glycosaminoglycanes de préférence acides ou leurs sels ou dérivés physiologiquement acceptables, notamment les acides hyaluroniques ou leurs sels (hyaluronates) physiologiquement acceptables, (ii) des polysaccharides et des hydrocolloïdes, en particulier les carragènes (carragénanes), (iii) des celluloses et des dérivés de la cellulose, en particulier les esters de cellulose et les éthers de cellulose, (iv) des acides alginiques et leurs sels (alginates), (v) des poly(acide (méth)acrylique) et des poly(méth)acrylates, (vi) des polyalkylèneglycols, en particulier les polyéthylèneglycols, (vii) des xanthanes et des combinaisons de deux ou plusieurs des composés mentionnés ci-dessus, de préférence dans le groupe des acides hyaluroniques ou leurs sels physiologiquement acceptables (hyaluronates), des carragènes (carragénanes) et leurs combinaisons.

9. Composition selon une quelconque des revendications précédentes,
la composition contenant également, en tant que composant (g), au moins l'ectoïne ou un dérivé d'ectoïne, en particulier une hydroxyectoïne, en particulier en quantités relatives situées dans la plage allant de 0,0001 à 10 % en poids, en particulier de 0,001 à 5 % en poids, de préférence de 0,01 à 2 % en poids, par rapport à la composition ; et / ou
la composition contenant en outre en tant que composant (h) au moins un alpha-sympathomimétique de préférence à base d'imidazoline ou ses sels physiologiquement acceptables ;
en particulier, dans laquelle l'alpha-sympathomimétique de préférence à base d'imidazoline est choisi parmi la xylométazoline ou l'oxymétazoline, notamment sous la forme de leurs sels physiologiquement acceptables, de manière davantage préférée sous la forme de leur chlorhydrate, et de manière particulièrement préférée, le chlorhydrate de xylométazoline ; et / ou
en particulier la composition contenant l'alpha-sympathomimétique de préférence à base d'imidazoline, par rapport à la composition, en des quantités relatives situées dans la plage allant de 0,001 à 2 % en poids, en particulier de 0,005 à 1,5 % en poids, de préférence de 0,01 à 1,2 % en poids, de préférence de 0,02 à 1,0 % en poids, de manière particulièrement préférée de 0,03 à 0,5 % en poids, de manière encore plus préférée de 0,04 à 0,2 % en poids ; et / ou
la composition contenant en outre du chlorure de sodium, en particulier en des quantités relatives situées dans la plage allant de 0,001 à 5 % en poids, en particulier de 0,01 à 2 % en poids, de préférence de 0,1 à 1 % en poids, sur la base de la composition

10. Composition selon une quelconque des revendications précédentes,
la composition contenant en association et pour chacun en des quantités efficaces, en particulier pharmaceutiquement efficaces :
(a) du cinéol, de préférence le 1,8-cinéol, en des quantités relatives situées dans la plage allant de 0,001 à 10 % en poids, en particulier de 0,002 à 5 % en poids, de préférence de 0,01 à 3 % en poids, de manière plus préférée de 0,05 à 3 % en poids, de manière particulièrement préférée de 0,1 à 2,5 % en poids, de manière tout particulièrement préférée de 0,2 à 2 % en poids, de manière encore plus préférée de 0,3 à 1 % en poids, idéalement de 0,4 à 0,8 % en poids ;
(b) du pantothénol, de préférence le dexpantothénol (D-pantothénol) ou ses esters physiologiquement acceptables, en des quantités relatives situées dans la plage allant de 0,01 à 10 % en poids, en particulier de 0,1 à 8 % en poids, de préférence de 0,5 à 7 % en poids, de manière plus préférée de 1 à 6,5 % en poids, de manière particulièrement préférée de 2 à 6 % en poids, de manière encore plus préférée de 4 à 6 % en poids, idéalement de 4,5 à 5,5 % en poids ;
(c) le cas échéant, au moins un émulsifiant, en particulier en des quantités relatives situées dans la plage allant de 0,0001 à 5 % en poids, en particulier de 0,001 à 2,5 % en poids, préférentiellement de 0,005 à 2 % en poids, de préférence de 0,01 à 1,5 % en poids, de manière plus préférée de 0,01 à 1 % en poids, de manière particulièrement préférée de 0,01 à 0,5 % en poids, de manière encore plus préférée de 0,02 à 0,08 % en poids ;
(d) le cas échéant, au moins un système tampon chimique, en particulier sous forme de sel (s) tampon(s), en particulier, en des quantités relatives, exprimées par la somme de tous les composants du système tampon chimique, situées dans la plage allant de 0,001 à 4 % en poids, en particulier de 0,01 à 3 % en poids, de préférence de 0,05 à 2 % en poids, de manière plus préférée de 0,1 à 1,5 % en poids, de manière particulièrement préférée de 0,2 à 1 % en poids ;
(e) le cas échéant, au moins un conservateur et / ou un désinfectant (antiseptique), en particulier en des quantités relatives situées dans la plage allant de 0,001 à 10 % en poids, en particulier de 0,005 à 5 % en poids, de préférence de 0,01 à 2 % en poids, de manière plus préférée de 0,01 à 1 % en poids, plus préférablement de 0,01 à 0,5 % en poids, encore plus préférablement de 0,02 à 0,1 % en poids ;
(f) le cas échéant, au moins un agent épaississant, en particulier en des quantités relatives situées dans la plage allant de 0,0001 à 10 % en poids, en particulier de 0,001 à 8 % en poids, de préférence de 0,005 à 5 % en poids, de manière plus préférée de 0,01 à 3 % en poids , plus préférablement de 0,05 à 2 % en poids, encore plus préférablement de 0,1 à 1 % en poids ;
(g) le cas échéant, au moins une ectoïne ou un dérivé d'ectoïne, en particulier une hydroxyectoïne, en particulier en des quantités relatives situées dans la plage allant de 0,0001 à 10 % en poids, en particulier de 0,001 à 5 % en poids, de préférence de 0,01 à 2 % en poids ;
(h) le cas échéant, au moins un alpha-sympathomimétique de préférence à base d'imidazoline ou ses sels physiologiquement acceptables, en particulier en des quantités relatives situées dans la plage allant de 0,001 à 2 % en poids, en particulier de 0,005 à 1,5 % en poids, de préférence de 0,01 à 1,2 % en poids, de manière plus préférée de 0,02 à 1,0 % en poids, de manière particulièrement préférée de 0,03 à 0,5 % en poids, le plus préférablement de 0,04 à 0,2 % en poids ;
(i) du chlorure de sodium, en particulier en des quantités relatives situées dans la plage allant de 0,001 à 5 % en poids, en particulier de 0,01 à 2 % en poids, de préférence de 0,1 à 1 % en poids ;
dans laquelle toutes les quantités relatives susmentionnées sont rapportées à la composition.

11. Composition selon une quelconque des revendications précédentes,
la composition étant présente sous la forme d'une composition aqueuse et/ou la composition ayant une base aqueuse et/ou se présentant sous la forme d'une formulation aqueuse, en particulier sous la forme d'une solution aqueuse ou d'une suspension aqueuse.

12. Composition selon une quelconque des revendications précédentes,
la composition ayant, à une température de 20° C et en l'absence d'un épaississant, une viscosité dynamique située dans la plage allant de 1,001 à 2 mPa.s, en particulier de 1,01 à 1,9 mPa.s, de préférence de 1,05 à 1,8 mPa.s, de manière plus préférée de 1,1 à 1,5 mPa.s (notamment déterminée par la méthode selon Ph. Eur. 7ème édition. ouvrage de base 2011, monographie 2.2.9 Viscosimètre capillaire) ; ou cependant
la composition a, à une température de 20°C et en présence d'un épaississant, une viscosité dynamique d'au moins 1,5 mPa.s, en particulier située dans la plage allant de 1,5 à 10⁵ mPa.s, de préférence de 1,6 à 10⁴ mPa.s, de manière plus préférée de 1,7 à 10³ mPa.s, plus préférentiellement de 2 à 100 mPa.s (en particulier déterminée par la méthode Ph. Eur. 7ème édition. ouvrage de base 2011, monographie 2.2.9 Viscosimètre capillaire).

13. Composition selon une quelconque des revendications précédentes,
la composition présentant au moins un autre ingrédient, en particulier un adjuvant et/ou un additif, en particulier choisi dans le groupe des auxiliaires de mise en oeuvre, des stabilisants, des émulsifiants, des antioxydants, des humectants, des épaississants, des antiseptiques, des colorants, des arômes, des substances odoriférantes, des parfums, des diluants, des liants, des agents mouillants, des vitamines, des oligo-éléments, des minéraux, des micronutriments et/ou des huiles essentielles et de leurs combinaisons.

14. Composition selon l'une quelconque des revendications précédentes,
destinée au traitement topique prophylactique et / ou curatif de la *Rhinitis acuta* (rhinite aiguë) ; ou
destinée au traitement topique prophylactique et / ou curatif des rhinites de toute nature, en particulier de la rhinite aiguë, de la rhinite allergique, de la rhinite atrophique, de la rhinite hyperplasique ou hypertrophique, de la rhinite mutilante, du rhume des foins ou rhinite vasomotrice, de la rhinite conditionnée par l'environnement ou de la rhinite pseudo-membraneuse, de préférence la rhinite aiguë.

15. Dispositif d'administration, en particulier pour l'administration topique, notamment nasale, de préférence intranasale, préférablement sous la forme d'un contenant doté d'un compte-goutte ou d'un pulvérisateur, contenant une composition selon l'une des revendications précédentes.
